Europäisches Patentamt

European Patent Office

Office européen des brevets

(19)

(11) EP 0 815 258 B1

(12) FASCICULE DE BREVET EUROPEEN

(45) Date de publication et mention
de la délivrance du brevet:
**06.10.1999 Bulletin 1999/40**

(21) Numéro de dépôt: **96908160.3**

(22) Date de dépôt: **20.03.1996**

(51) Int Cl.⁶: **C12Q 1/04**, C12Q 1/24

(86) Numéro de dépôt international:
**PCT/FR96/00419**

(87) Numéro de publication internationale:
**WO 96/29427 (26.09.1996 Gazette 1996/43)**

(54) **Procédé de détection de microorganismes par séparation et culture sur un système gélifié, système gélifié et nécessaire de dosage pour mettre en oeuvre ce procédé, utilisation en microbiologie**

Verfahren zum Nachweis von Mikroorganismen durch Trennung und Kultivierung auf einem Gelsystem, Gelsystem und Testsatz zur Durchführung dieses Verfahrens

Method for detecting microorganisms by separation and culture on a gelled system, gelled system and assay kit therefor, and use thereof in microbiology

(84) Etats contractants désignés:
**AT BE CH DE DK ES FI FR GB GR IE IT LI LU MC NL PT SE**

(30) Priorité: **20.03.1995 FR 9503214**

(43) Date de publication de la demande:
**07.01.1998 Bulletin 1998/02**

(73) Titulaire: **DIFFUSION BACTERIOLOGIE DU VAR 83870 Signes (FR)**

(72) Inventeurs:
• **CONTANT-PUSSARD, Geneviève F-92400 Courbevoie (FR)**
• **BOISARD-BEAUPERE, Françoise F-75017 Paris (FR)**

• **MARTINOLI, Jean-Luc F-92390 Villeneuve-la-Garenne (FR)**
• **ROUSSEAU, Alain F-75004 Paris (FR)**

(74) Mandataire: **Clisci, Serge et al S.A. FEDIT-LORIOT & AUTRES CONSEILS EN PROPRIETE INDUSTRIELLE 38, Avenue Hoche 75008 Paris (FR)**

(56) Documents cités:
**EP-A- 0 454 509          WO-A-91/04318
US-A- 3 932 222           US-A- 4 164 449
US-A- 4 190 328           US-A- 4 212 948
US-A- 4 717 660**

## Description

### *Domaine de l'invention*

La présente invention a trait à un nouveau procédé pour détecter la présence ou l'absence de microorganismes appartenant à l'ensemble des bactéries et des levures. Ce procédé met en oeuvre une technique de séparation, de culture et de révélation sur un même système gélifié.

Elle concerne également ledit système gélifié en tant que produit industriel nouveau, d'une part, et le nécessaire, kit ou trousse de dosage permettant de mettre en oeuvre ledit procédé, d'autre part.

Elle concerne enfin l'utilisation dudit procédé et dudit système gélifié en microbiologie (notamment en bactériologie ou mycologie), en particulier dans le domaine de la détection des infections bactériennes (plus particulièrement celle des infections urinaires et des septicémies).

### *Art antérieur*

Quand on veut apprécier la présence ou l'absence de microorganismes tels que les bactéries et les levures, notamment selon une méthode colorimétrique, on doit tenir compte des résultats suivants :

- les vrais positifs (TP),
- les faux positifs (FP),
- les vrais négatifs (TN), et
- les faux négatifs (FN).

D'un point de vue pratique, à partir des résultats TP, FP, TN et FN théoriquement possibles, quatre valeurs (exprimées en pourcentages) ont été définies, à savoir :

- la sensibilité (Sen),
- la spécificité (Spe),
- la valeur prédictive positive (PPV), et
- la valeur prédictive négative (NPV),

telles que :

$$Sen = 100.TP/(TP+FN),$$

$$Spe = 100.TN/(TN+FP),$$

$$PPV = 100.TP/(TP+FP),$$

et

$$NPV = 100.TN/(TN+FN),$$

pour évaluer le caractère significatif ou la pertinence des mesures.

On sait que l'on a déjà préconisé ou utilisé une technique de détection ou de filtration colorimétrique pour apprécier la présence ou l'absence de bactéries dans l'urine, d'une part, et une technique de séparation sur gel pour mettre en évidence des agglutinats érythrocytaires, d'autre part.

En ce qui concerne la détection colorimétrique, on connaît de la publication EP-A-0 496 409 un procédé de révélation de bactéries Gram-négatif. Ce procédé comprend la filtration d'un échantillon biologique (notamment l'urine) contenant des bactéries sur un support poreux (diamètre des pores 0,75-1,2 μm) pour retenir les bactéries sur ledit support, puis la détection des bactéries Gram-négatif par la réaction de leurs déhydrogénases avec un réactif coloré d'oxydo-réduction (notamment un sel de tétrazolium ou la résazurine) en inhibant au moyen d'un agent caotrope et d'un détergent non-ionique du type alkyl-glucoside les réactions de réduction induites par les bactéries Gram-positif.

En ce qui concerne la technique de filtration colorimétrique, on connaît notamment des tests commercialisés par la société dite VITEK SYSTEMS (Hazlewood, Mo.) sous les nomenclatures de "Bac-T-Screen®" (test semi-automatique) et de "FiltraCheck®-UTI" (test manuel).

La mise en oeuvre de ces tests comprend la filtration d'un échantillon d'urine sur un filtre en papier imprégné d'un révélateur pour retenir et colorer les bactéries éventuellement présentes dans l'échantillon ; la filtration est réalisée avec une différence de pression, aussi un appareillage relativement compliqué est requis ; la principale source d'erreurs (résultats FP et FN) est due à la présence possible dans l'urine d'un grand nombre d'érythrocytes, de leucocytes et/ou de cellules épithéliales (la présence d'érythrocytes et/ou de leucocytes dans l'urine est déjà en soi un signe de dysfonctionnement).

Selon la littérature, voir à cet effet (a) les pages 272 (tableau 3) et 274 (chapitre "Colorimetric Filtration") de l'article de M. PEZZLO, *Clin. Microbiol. Rev.*, 1988, 1 (No 2), 268-280, et (b) la page 86 (alinéa "Système coloration filtration" et tableaux) de l'article de F.W. GOLDSTEIN, *Méd. Mal. Infect.*, 1991, 21, 83-88, les tests Bac-T-Screen® et FiltraCheck®-UTI sont (i) rapides (1-2 minutes), (ii) efficaces quand la population bactérienne est $\geq 10^5$ CFU/ml, mais (iii) insatisfaisants quand ladite population bactérienne est inférieure à $10^5$ CFU/ml. Globalement pour l'ensemble de ces deux tests on a selon les deux articles précités, lors de screenings urinaires, pour des populations bactériennes $\geq 10^5$ CFU/ml: Sen = 93-95%, Spe = 76-77%, PPV = 32%, et NPV = 97-99% ;

$\geq 10^4$ CFU/ml : Sen = 85-89%, Spe = 81%, et NPV = 84-86% ; et

$\geq 10^3$ CFU/ml : Sen = 76-84%, et NPV = 67-72%.

Il existe donc un besoin d'améliorer la fiabilité des mesures pour des populations bactériennes inférieures à $10^5$ CFU/ml.

En ce qui concerne la technique de séparation sur gel des érythrocytes pour apprécier l'agglutination érythrocytaire on connaît les publications FR-A-2 577 321 et EP-A-0 454 509.

Selon FR-A-2 577 321, la formation ou la présence d'agglutinats érythrocytaires sont mises en évidence par (1) dépôt d'un milieu liquide contenant des érythrocytes sur un gel (i) disposé dans un tube de centrifugation à fond conique, et (ii) laissant passer les globules libres plus facilement que les agglutinats [notamment un gel commercialisé sous le nom de "Sephadex® G 100 Ultrafine" par la société dite PHARMACIA FINE CHEMICAL (Uppsala, Suède)], puis (2) centrifugation douce.

Selon EP-A-0 454 509, on opère de façon sensiblement analogue par centrifugation douce en remplaçant le gel de FR-A-2 577 321 par un système gélifié constitué par un mélange de deux gels de dextrane (voir notamment page 3 lignes 2-4 et page 4 lignes 6-7 de EP-A-0 454 509) : le produit "Sephadex® G 100 Ultrafine" précité qui, à l'état sec, se présente sous la forme de particules ayant un diamètre de 20 à 50 μm, et le produit : "Sephadex® HP Ultrafine" qui, à l'état sec, se présente sous la forme de particules ayant un diamètre de 13 à 23 μm.

Eu égard aux indications fournies dans EP-A-0 454 509 (page 3 lignes 2-4 et 42-43, et page 4 lignes 14-15), il semble clair que le système gélifié de EP-A-0 454 509 ne comporte qu'une seule phase.

Telle que décrite dans FR-A-2 577 321 et EP-A-0 454 509, la technique de séparation sur gel par centrifugation n'est pas directement transposable à la détection de la présence ou l'absence de microorganismes. En particulier les gels préconisés, à savoir les produits "Sephadex® G 100 Ultrafine" et "Sephadex® HP Ultrafine", ne permettent pas le passage des microorganismes tels que les bactéries et les levures lors de la centrifugation douce [i.e. selon l'art antérieur : une intensité inférieure ou égale à 1500 g, en particulier une intensité inférieure à 1000 g (notamment de l'ordre de 400-500 g, EP-A-0 454 509 signalant, page 4 ligne 49, une intensité de 430 g)] ni la séparation des bactéries des autres cellules.

### But de l'invention

Selon l'invention on se propose de fournir une nouvelle solution technique pour détecter dans un échantillon d'un matériau biologique la présence ou l'absence de microorganismes appartenant à l'ensemble des bactéries et des levures, cette nouvelle solution technique donnant des résultats plus fiables pour des populations microbiennes inférieures à 105 germes/ml (dans ce qui suit on considérera par commodité que 1 bactérie/ml ou 1 levure/ml correspond approximativement à 1 CFU/ml) que les tests "Bac-T-Screen®" et "FiltraCheck®-UTI" précités relatifs à l'évaluation des infections urinaires bactériennes.

Cette nouvelle solution technique met en oeuvre une technique particulière de séparation des microorganismes sur un système gélifié contenant un milieu de culture, qui est différente de celle proposée par les publications FR-A-2 577 321 et EP-A-0 454 509 précitées, et qui évite de mettre en oeuvre l'isolement des microorganismes sur un système gélosé standard ou la mise en culture nécessaire à leur multiplication, avant centrifugation.

### Objet de l'invention

Ce but est obtenu en faisant appel à un système gélifié qui sépare sélectivement les microorganismes éventuellement présents dans un échantillon d'un matériau biologique des autres composants notamment les cellules, les peptides et les protéines, d'une part, et qui permet la pousse desdits microorganismes in situ pendant la centrifugation puis le cas échéant après ladite centrifugation et leur révélation, d'autre part.

Selon un premier aspect de l'invention, on préconise un nouveau procédé de détection de la présence ou de l'absence de microorganismes appartenant à l'ensemble des bactéries et des levures dans un échantillon liquide d'un matériau biologique susceptible de contenir des cellules autres que celles desdits microorganismes, ledit procédé, qui met en oeuvre une technique de séparation sur gel, étant caractérisé en ce qu'il comprend les étapes consistant à :

(1°) faire appel à un système gélifié qui (i) sépare en fonction de leur taille les microorganismes, éventuellement présents dans ledit échantillon liquide et qui proviennent dudit matériau biologique, des autres composants solides que peut contenir ledit échantillon liquide, (ii) est essentiellement imperméable à l'eau pouvant être présente dans ledit échantillon liquide et aux substances dissoutes éventuellement présentes dans ledit échantillon liquide, et (iii) comporte au moins

(a) une première phase, dite de révélation, qui est un gel comportant un milieu de culture de microorganismes et un réactif induisant une variation de mesure optique détectable en présence de microorganismes, ledit gel étant un mélange intime d'eau et de particules polymériques absorbant l'eau, qui ont été gonflées de telle façon que dans ledit mélange intime lesdites particules polymériques aient une concentration en poids sec comprise entre 0,05 et 0,2 g/ml et un diamètre à l'état gonflé compris entre 90 et 320 µm, l'eau dudit mélange intime provenant en totalité ou en partie dudit milieu de culture ;

(2°) introduire ledit échantillon liquide dans un tube de centrifugation au-dessus dudit système gélifié préalablement disposé dans ledit tube de centrifugation ;
(3°) centrifuger le contenu dudit tube résultant ; et,
(4°) révéler la présence ou l'absence de microorganismes dans ledit échantillon liquide provenant dudit matériau biologique, au niveau de ladite première phase du système gélifié, par ledit réactif induisant une variation de mesure optique détectable.

Si les microorganismes sont à une concentration trop faible dans ledit échantillon liquide, la révélation de l'étape (4°) intervient après multiplication des microorganismes dans ledit milieu de culture, à une température comprise entre la température ambiante et 45°C, en au plus 24 h (de préférence en 1-2 h) pour les bactéries et en au plus 36 h (de préférence en 24-30 h) pour les levures.

Selon un second aspect de l'invention, on préconise, en tant que produit industriel nouveau, ledit système gélifié qui (i) sépare en fonction de leur taille les microorganismes, éventuellement présents dans ledit échantillon liquide et qui proviennent dudit matériau biologique, des autres composants solides que peut contenir ledit échantillon liquide, (ii) est essentiellement imperméable à l'eau pouvant être présente dans ledit échantillon liquide, et (iii) comporte au moins

(a) une première phase, dite de révélation, qui est un gel comportant un milieu de culture de microorganismes et un réactif induisant une variation de mesure optique détectable en présence de microorganismes, ledit gel étant un mélange intime d'eau et de particules polymériques absorbant l'eau, qui ont été gonflées de telle façon que dans ledit mélange intime lesdites particules polymériques aient une concentration en poids sec comprise entre 0,05 et 0,2 g/ml et un diamètre à l'état gonflé compris entre 90 et 320 µm, l'eau dudit mélange intime provenant en totalité ou en partie dudit milieu de culture.

Selon encore un autre aspect de l'invention, on préconise un nécessaire, kit ou trousse de dosage comprenant notamment :

- ledit système gélifié ou les phases le constituant, et
- le cas échéant, des souches de microorganismes de référence lyophilisées et/ou leurs milieux de culture pour permettre de réaliser des échantillons-étalons.

Selon enfin un autre aspect de l'invention, on préconise l'utilisation dudit procédé et dudit système gélifié en microbiologie, notamment dans (i) la détection des infections bactériennes (en particulier telles que les infections urinaires et les septicémies), (ii) l'identification des souches de microorganismes, et (iii) l'évaluation de la résistance de souches de microorganismes vis-à-vis des antibiotiques.

**Brève description des dessins**

Les dessins joints en annexe représentent de façon schématique des tubes de centrifugation garnis chacun d'un système gélifié selon l'invention :

- la figure 1 représente un tube de centrifugation, comportant un système gélifié constitué d'une seule phase, à savoir la phase dite de révélation ;
- les figures 2a et 2b représentent un tube de centrifugation comportant un système gélifié constitué de deux phases qui sont, du bas vers le haut, la phase de révélation et la phase de protection, et montré avant (Fig. 2a) et après (Fig. 2b) centrifugation ;
- la figure 3 représente un tube de centrifugation, comportant un système gélifié constitué de deux phases qui sont, du bas vers le haut la phase de révélation et la phase d'absorption ;
- les figures 4a et 4b représentent un système gélifié analogue à celui de la figure 3, qui comporte en outre la phase de protection et est vu avant (figure 4a) et après (figure 4b) centrifugation ;
- la figure 5 représente un tube de centrifugation, comportant un système gélifié constitué de trois phases qui sont du bas vers le haut la phase barrière, la phase de révélation et la phase d'absorption ;
- les figures 6a et 6b représentent un système gélifié analogue à celui de la figure 5, qui comporte en outre la phase de protection et qui est montré avant (figure 6a) et après (figure 6b) centrifugation ;
- les figures 7 et 8 représentent chacun un système gélifié préféré selon l'invention où la phase de révélation a une épaisseur faible ;
- la figure 9 représente une coupe d'un tube de centrifugation selon l'invention pourvu à son fond d'un capillaire ; et,
- la figure 10 représente une vue de face d'un autre tube de centrifugation selon l'invention qui est également pourvu d'un capillaire.

**Abréviations**

Par commodité, les abréviations et acronymes suivants ont été utilisés dans le texte de la présente invention :

Ala     alanyle
BT      bleu de tétrazolium
CFU     unité formant colonie (en anglais : "colony-forming unit")
DIC     coagulation intravasculaire disséminée
EDTA    acide éthylènediaminotétraacétique
FN      faux négatifs (en anglais : "false negatives")
FP      faux positifs (en anglais : "false positives")
Hyp     hydroxyprolyle, Hyp représente 3Hyp (3-hydroxyprolyle) et/ou 4Hyp (4-hydroxyprolyle)
INT     iodonitrotétrazolium
Leu     leucyle
Lys     lysyle
MA      4-méthylcoumarinyl-7-amino
MUG     4-méthylumbelliféryl-$\beta$-D-glucuronide
MW      poids moléculaire
NA      $\beta$-naphtylamino
NADH    nicotinamide-adénine dinucléotide hydrogéné
NBT     nitrobleu de tétrazolium
NPV     valeur prédictive négative (en anglais : "negative predictive value")
NTC     chlorure de néotétrazolium
OM      mesure optique
Phe     $\alpha$-phénylalanyle
Phg     $\alpha$-phénylglycyle
pNA     p-nitroanilino
PPV     valeur prédictive positive (en anglais : "positive predictive value")
Pro     prolyle
RT      température ambiante (15-20°C)
Sen     sensibilité (en anglais : "sensitivity")
Spe     spécificité (en anglais : "specificity")
TN      vrais négatifs (en anglais : "true negatives")
TP      vrais positifs (en anglais : "true positives")

TTC    chlorure de triphényltétrazolium
YNB    source d'azote commercialisée sous la nomenclature commerciale "YEAST NITROGEN BASE"

### Description détaillée de l'invention

Par "matériau biologique" on entend ici :

- un liquide corporel humain ou animal, qui est aqueux, notamment l'urine, le sang, le plasma, le liquide rachidien, le liquide pleural, le lait ;
- une eau quelconque, notamment l'eau vive (sources, rivières), l'eau stagnante (mares, réservoirs, piscines), l'eau industrielle (eau du robinet, eau des canalisations et circuits intervenant en particulier en tant que fluide thermique ou énergétique, eau usée), l'eau de boisson ;
- une boisson, notamment d'origine agro-alimentaire et à base d'eau telle que les jus de fruits, les sodas, le lait précité, le vin, la bière ;
- un aliment d'origine animale et/ou végétale ou une préparation alimentaire ;
- un extrait de plante notamment un extrait aqueux, alcoolique ou hydroalcoolique, en particulier un jus de pression ou un extrait aqueux ;
- un produit solide ou visqueux, tel que notamment le pus, les biopsies, les fèces, le crachat, la salive, une plante, une partie de plante, un sol, le sable, ledit produit pouvant contenir ou non de l'eau dans sa composition ; et,
- d'une manière générale, l'environnement de l'homme (notamment le sol et l'eau précités, d'une part, et l'atmosphère ambiante, d'autre part) ; des exemples sont donnés ci-après pour l'évaluation de la qualité bactériologique et mycologique de l'atmosphère des blocs opératoires.

En bref, ledit matériau biologique sera choisi parmi l'ensemble constitué par les liquides corporels, les aliments, les boissons et les produits de l'environnement (y compris les plantes et leurs extraits).

En pratique, il est recommandé mais non essentiel que ledit matériau biologique soit un produit aqueux ; il suffit pour mettre en oeuvre le procédé de détection de l'invention de façon optimale que ledit matériau biologique soit transformé (notamment par broyage, dilacération, extraction et/ou addition d'eau) en un échantillon liquide ou soit déjà sous forme liquide pour être testé. De façon avantageuse ledit échantillon liquide sera un échantillon aqueux liquide.

Par "cellule" on entend ici toute cellule biologique qui n'est pas une bactérie ni une levure. Par "matériau cellulaire" on entend ici un matériau choisi parmi (i) les cellules, (ii) les fragments desdites cellules (notamment les fragments de paroi cellulaire), (iii) le contenu desdites cellules logé à l'intérieur de la paroi et (iv) leurs mélanges.

Le matériau biologique selon l'invention, dans lequel on veut déterminer la présence ou l'absence de microorganismes, et son échantillon liquide peuvent contenir un matériau cellulaire.

A titre d'information, l'urine d'un patient peut contenir :

(A) des microorganismes en cas d'infection urinaire, tels que :

- les bactéries Gram-négatif, notamment les **Escherichia coli, Klebsiella** (en particulier **Klebsiella aerogenes, Klebsiella pneumoniae), Proteus** (en particulier **Proteus vulgaris, Proteus mirabilis), Pseudomonas** (en particulier **Pseudomonas aeruginosa), Serratia** (en particulier **Serratia marcescens)** et/ou **Enterobacter** (en particulier **Enterobacter aerogenes),**
- les bactéries Gram-positif, notamment les **Staphylococcus** (en particulier **Staphylococcus aureus, Staphylococcus epidermidis, Staphylococcus saprophyticus), Streptococcus** (en particulier **Streptococcus faecalis, Streptococcus faecium), Enterococcus** et/ou **Corynebacterium,** et/ou
- les levures notamment les **Candida albicans, Candida glabrata** (ancienne nomenclature : **Torulopsis glabrata), Candida tropicalis** et/ou **Candida krusei,**

qui sont susceptibles d'être pathogènes (ces souches peuvent induire des septicémies et des DIC), d'une part ; et,
(B) un matériau cellulaire comprenant des cellules (en particulier érythrocytes, leucocytes et/ou cellules épithéliales), des enzymes cellulaires (en particulier urokinase urinaire, leucocyte estérase et/ou phosphatase), des antibiotiques (pouvant perturber ou fausser la détection des microorganismes présents dans l'urine), des peptides, des protéines, des pigments et/ou des fragments de paroi cellulaire, d'autre part.

Selon l'invention, on va faire migrer, à travers le système gélifié, les particules non dissoutes dans l'échantillon liquide dudit matériau biologique, de façon à séparer en fonction de leur taille les microorganismes des autres composants solides et non dissous provenant dudit matériau biologique par centrifugation, d'une part, puis on va en général faire pousser *in situ* lesdits microorganismes, d'autre part, afin de les détecter.

Par "réactif induisant une variation de mesure optique détectable" (autre nomenclature : "réactif colorimétrique"), on entend ici un produit qui, en présence d'un microorganisme appartenant à l'ensemble des bactéries et des levures, provoque une variation de OM appréciée soit à l'oeil nu soit au moyen d'un colorimètre. En d'autre terme le réactif changeant de couleur en présence de microorganismes induit un changement de couleur dans le spectre visible ou un changement de fluorescence ou de luminescence notamment dans le domaine UV.

Un tel "réactif colorimétrique" peut être (i) un indicateur coloré de pH, tel que le rouge de phénol, (ii) un indicateur coloré d'oxydoréduction, tel que notamment le dichloroindophénol, la résazurine ou un sel de tétrazolium (par exemple BT, INT, NBT, NTC ou TTC), (iii) un substrat chromogène, tel que H-L-Ala-pNA, H-L-Leu-pNA, H-L-Phe-pNA, H-L-Pro-pNA, H-L-Lys-pNA, H-L-Hyp-pNA, un substrat fluorogène, tel que H-L-Ala-NA, H-L-Leu-NA, H-L-Phe-NA, H-L-Pro-NA, H-L-Lys-NA, H-L-Hyp-NA, H-L-Ala-MA, H-L-Leu-MA, H-L-Phe-MA, H-L-Pro-MA, H-L-Lys-MA, H-L-Hyp-MA, MUG phosphate, (iv) un substrat clivable par une osidase des microorganismes telle que la β-D-glucosidase ou mieux la β-D-galactosidase, ou (v) un substrat chimioluminescent, tel un composé de luciférine.

Les indicateurs colorés d'oxydo-réduction sont principalement des composés oxydants qui révèlent la présence de microorganismes par réaction avec au moins une substance réductrice provenant desdits microorganismes, telle que notamment NADH et les déshydrogénases. Le mécanisme est analogue à celui illustré ci-après pour la résazurine :

$$\text{résazurine} + \text{NADH} \rightarrow \text{résorufine} + \text{NAD}^+$$

Les substrats chromogènes et fluorogènes que l'on préfère selon l'invention, à savoir H-L-Ala-pNA, H-L-Leu-pNA, H-L-Phe-pNA, H-L-Pro-pNA, H-L-Lys-pNA, H-L-Hyp-pNA, H-L-Ala-NA, H-L-Leu-NA, H-L-Phe-NA, H-L-Pro-NA, H-L-Lys-NA et H-L-Hyp-NA sont clivés par les aminopeptidases produites par les microorganismes. D'autres substrats peptidiques clivables par lesdites aminopeptidases, qui sont décrits dans la demande PCT publiée WO-A-90/03726, sont également utilisables dans la présente invention.

Comme indiqué ci-dessus le système gélifié selon l'invention, qui intervient à l'étape (1°) du procédé préconisé, comporte au moins une couche gélifiée, à savoir la "phase de révélation" également désignée ici par commodité "première phase" ou "première couche". La phase de révélation est constituée d'une substance polymérique (notamment du type dextrane ou analogue) préalablement gonflée avec de l'eau de façon à former un gel retenant dans sa masse (ou au niveau de sa face inférieure pour les plus grosses particules), lors de la centrifugation, les particules non solubles contenues dans ledit échantillon liquide et ayant une taille se situant entre 0,5 et 8 μm.

Il est important que, à l'état gonflé, ladite substance polymérique de la phase de révélation soit à une concentration en poids sec de 0,05 à 0,2 g/ml (de préférence à une concentration de 0,1 g/ml) et se présente (dans le gel) sous la forme de particules gonflées ayant un diamètre de 90 à 320 μm, pour retenir dans sa masse ou au voisinage de sa face inférieure les bactéries (longueur : 0,5 à 5 μm ; diamètre : 0,1 à 2 μm) et la majeure partie des levures (diamètre : 5 à 12 μm) mais ne pas retenir les cellules.

De façon pratique, la substance polymérique de la phase de révélation sera préalablement gonflée avec le milieu de culture destiné à la pousse des microorganismes, ledit milieu de culture contenant le réactif colorimétrique précité induisant une variation de OM.

Le système gélifié selon l'invention peut en outre comporter pour la mise en oeuvre de l'étape (1°) du procédé préconisé

(b) une seconde phase, dite d'absorption, essentiellement imperméable à l'eau pouvant être présente dans ledit échantillon liquide et aux substances qui y sont dissoutes, qui retient dans sa masse les particules non solubles contenues dans ledit échantillon liquide et ayant une taille inférieure à 5 μm mais laisse passer les particules ayant une taille supérieure ou égale à 5 μm lors de la centrifugation, et qui est constituée (i) d'un gel ou (ii) de particules de silice ou de silicate ayant une granulométrie inférieure à 0,1 μm, ladite seconde phase reposant sur ladite première phase, ledit mélange intime étant essentiellement imperméable à l'eau pouvant être présente dans ledit échantillon liquide.

Selon une variante de réalisation, la "phase d'absorption" ou "deuxième phase" ou encore "deuxième couche" est un gel constitué par un mélange intime d'eau et de particules polymériques absorbant l'eau, qui ont été gonflées de telle façon que dans ledit mélange intime lesdites particules polymériques aient (α) une concentration en poids sec comprise entre 0,2 et 1 g/ml et (β) un diamètre à l'état gonflé compris entre 160 et 530 μm.

Dans ce cas la phase d'absorption est constituée d'une substance polymérique (notamment du type dextrane ou analogue) préalablement gonflée avec de l'eau de façon à former un gel, comme ladite première phase, mais de façon à retenir dans sa masse (ou au niveau de sa face inférieure pour les plus grosses particules), lors de la centrifugation, les particules non solubles contenues dans ledit échantillon liquide et ayant une taille plus faible se situant entre 0,1 et 0,5 µm, d'une part, et les particules non solubles ne traversant pas ladite première phase (i.e. taille inférieure à 5 µm), d'autre part.

Selon une autre variante, la phase d'absorption est une couche particulaire constituée d'une matière minérale, micronisée de façon à avoir à l'état sec une granulométrie inférieure à 0,1 µm, d'une part, et ne gonflant essentiellement pas en présence d'eau, d'autre part. Cette matière minérale sera avantageusement choisie parmi l'ensemble constitué par la silice (notamment le sable, le quartz ou le verre) et les silicates tels que les aluminosilicates. Parmi les silicates, on recommande plus particulièrement le kaolin qui est une argile non gonflante en présence d'eau.

On a en effet constaté que les substances minérales telles que la silice et le kaolin ayant une granulométrie inférieure à 0,1 µm permettent le passage des microorganismes et retiennent l'eau ainsi que les substances dissoutes susceptibles d'être présentes dans ledit échantillon liquide.

Ainsi la phase d'absorption va empêcher le passage des particules solides non dissoutes dans ledit échantillon liquide telles que l'hémoglobine, les pigments (notamment les pigments urinaires quand on cherche à détecter la présence ou l'absence de microorganismes dans l'urine en cas d'infection urinaire), les enzymes, les inhibiteurs, et va retenir les particules non dissoutes comme les fragments de cellules lysées. A titre d'exemple, ces fragments sont retenus après traitement avec un agent lyrique ou en présence d'un agent lytique. Cet agent lytique peut être un détergent tel que la saponine pour la lyse des érythrocytes.

Il est important que, (1) ladite substance polymérique de la phase d'absorption, à l'état gonflé, soit à une concentration en poids sec de 0,2 à 1 g/ml (de préférence à une concentration de 0,35 g/ml) et se présente (dans le gel) sous la forme de particules gonflées ayant un diamètre de 160 à 530 µm, et que (2) les particules minérales telles que la silice et le kaolin micronisés aient un diamètre homogène.

Quand elle est présente dans ledit système gélifié, ladite seconde phase renferme avantageusement une ou plusieurs substances inhibant les éventuels contaminants ou lysant les cellules (notamment les érythrocytes et/ou les leucocytes) et/ou éliminant les lipides (cas de la clarification des échantillons de lait à tester), qui sont contenus dans ledit échantillon liquide provenant dudit matériau biologique et susceptibles d'interférer à l'étape (4°) en réagissant avec ledit réactif induisant une variation de mesure optique détectable en présence de microorganismes.

Quand ladite seconde phase n'est pas présente dans le système gélifié de l'invention, il est recommandé que la ou lesdites substances (inhibitrices, lysantes et/ou éliminantes) soient incluses dans ladite première phase.

Le système gélifié selon l'invention peut en outre comporter pour la mise en oeuvre de l'étape (1°) du procédé préconisé

(c) une troisième phase, dite barrière, infranchissable lors de la centrifugation de l'étape (3°), par les microorganismes éventuellement présents, ladite troisième phase étant disposée sous la première phase de telle façon que ladite première phase repose sur ladite troisième phase.

Ladite troisième phase (ou "troisième couche") est soit une paraffine soit un gel d'une substance polymérique. Quand elle est une paraffine, il s'agit d'une paraffine solide fondant à une température supérieure à la température de multiplication des microorganismes, par exemple :

- une paraffine fondant à une température supérieure ou égale à 30°C, quand la culture pour la pousse des microorganismes intervenant à l'étape (4°) est réalisée à RT,
- une paraffine fondant à une température supérieure ou égale à 45°C, quand la culture pour la pousse des microorganismes intervenant à l'étape (4°) est réalisée à 37°C, ou
- une paraffine fondant à une température supérieure ou égale à 50-55°C, quand la culture pour la pousse des microorganismes intervenant à l'étape (4°) est réalisée à 45°C.

De préférence, on fera appel à une paraffine fondant à une température supérieure ou égale à 50°C qui sera introduite à l'état fondu dans le tube de centrifugation.

Quand la phase barrière est un gel, ledit gel sera constitué par un mélange intime d'eau et de particules polymériques absorbant l'eau, qui ont été gonflées de telle façon que dans ledit mélange intime lesdites particules polymériques aient (α) une concentration en poids sec comprise entre 0,04 et 0,2 g/ml et (β) un diamètre à l'état gonflé compris entre 30 et 130 µm.

Ledit gel est constitué d'une substance polymérique (notamment du type dextrane ou analogue) préalablement gonflée avec de l'eau de façon à former un gel, comme ladite première phase, mais de façon à retenir dans sa masse ou au niveau de sa face supérieure, lors de la centrifugation, les particules non solubles, contenues dans ledit échantillon liquide et ayant une taille plus importante (i.e. taille supérieure ou égale à 5 ou 8 µm).

Ainsi la phase barrière va retenir à sa surface supérieure (cas de la paraffine solide) ou dans sa masse (cas du gel) les cellules, notamment les érythrocytes ayant en moyenne une taille de 5-8 µm, les leucocytes ayant en moyenne une taille de 10-12 µm, les cellules épithéliales (en particulier dans le cas de l'urine) ayant en moyenne une taille de 20-50 µm, les cylindres ayant en moyenne une taille supérieure à 40 µm, les cristaux ayant en moyenne une taille de 20-200 µm, les fragments de paroi cellulaire (notamment, lorsque les cellules auront été lysées lors de la traversée, durant la centrifugation, de la première phase ou de l'ensemble deuxième phase/première phase).

De façon pratique, quelle que soit la nature de la phase barrière, les particules solides contenues dans ledit échantillon liquide vont se répartir selon un gradient : les plus petites (i.e. les moins lourdes d'entre elles) au-dessus des plus grandes (i.e. les plus lourdes d'entre elles).

Quand le système gélifié ne comporte pas la phase barrière, il est important que ledit système gélifié soit disposé dans un tube de centrifugation à fond conique (comme envisagé dans les documents FR-A-2 577 321 et EP-A-0 454 509 précités) ou à fond pourvu d'un capillaire. Dans ce cas, la répartition des particules solides les plus lourdes est réalisée au fond du tube de centrifugation comme indiqué ci-dessus, les microorganismes étant disposés juste au-dessus des cellules.

Il est important que, à l'état gonflé, ladite substance polymérique du gel de la phase barrière soit à une concentration en poids sec de 0,04 à 0,2 g/ml (de préférence à une concentration de 0,14 g/ml) et se présente sous la forme de particules gonflées ayant un diamètre de 30 à 130 µm.

Quand la phase barrière est présente dans le système gélifié selon l'invention, ledit système gélifié peut être logé dans un tube de centrifugation quelconque, notamment un tube à fond rond ou sphérique.

La présence de la phase d'absorption est avantageuse en ce sens qu'elle permet d'éviter que le liquide (principalement l'eau) dudit échantillon liquide soit en contact avec la phase de révélation avant et après la centrifugation.

La présence simultanée de la phase d'absorption et de la phase barrière est avantageuse en ce sens qu'elle autorise l'utilisation d'une phase de révélation de faible épaisseur. Par suite les microorganismes présents dans la masse ou au niveau de la face inférieure de ladite phase de révélation seront plus concentrés dans un volume donné et par suite plus facilement détectables lors de la révélation colorimétrique (soit à l'oeil nu soit au moyen d'un colorimètre).

Le système gélifié selon l'invention qui comprend ladite première phase seule ou associée avec au moins l'une desdites seconde phase et troisième phase est imperméable au liquide dudit échantillon liquide. Lors de la centrifugation, ledit liquide et les ingrédients qui y sont dissous ne traversent pas ledit système gélifié, en revanche les ingrédients non dissous dans ledit échantillon liquide migrent dans l'épaisseur dudit système gélifié et sont séparés en fonction de leurs tailles comme expliqué ci-dessus.

Le système gélifié selon l'invention peut en outre comporter pour la mise en oeuvre de l'étape (1°) du procédé préconisé

(d) une quatrième phase, dite de protection, qui (i) est une huile inerte de densité inférieure à celle de l'eau, de préférence une huile de paraffine, (ii) avant centrifugation, repose sur ladite première phase en l'absence de la seconde phase, ou ladite seconde phase quand celle-ci est présente dans ledit système gélifié, et (iii) après centrifugation selon l'étape (3°), repose sur l'eau dudit échantillon liquide.

L'huile de la phase de protection est nécessairement inerte vis-à-vis des constituants du système gélifié et des composants de l'échantillon liquide. On recommande plus particulièrement une huile qui fond à une température inférieure à RT et qui a un point d'ébullition supérieur à 50°C voire supérieur à 55°C. Convient en particulier une huile de paraffine. De façon pratique, on introduira un volume de 0,25 à 1 ml de la phase de protection dans un tube de centrifugation de 5 ml.

Selon l'invention, la phase de protection remplit avantageusement deux fonctions : elle évite la déshydratation et la contamination du système gélifié, lors du stockage, d'une part, et elle permet de ne pas dégrader, lors de l'introduction dudit échantillon liquide dans le tube de centrifugation par coulée, la couche dudit système gélifié sur laquelle elle repose, d'autre part.

On recommande de façon préférée, à l'étape (1°) de faire appel soit à un système gélifié comprenant, de haut en bas, dans un tube de centrifugation à fond conique :

- ladite quatrième phase,
- ladite seconde phase, et
- ladite première phase,

soit à un système gélifié comprenant, de haut en bas, dans un tube de centrifugation à fond rond ou sphérique :

- ladite quatrième phase,
- ladite seconde phase,
- ladite première phase, et
- ladite troisième phase,

soit à un système gélifié comprenant, de haut en bas, dans un tube de centrifugation dont le fond est un capillaire :

- le cas échéant, ladite quatrième phase et/ou, ladite seconde phase, et
- dans le capillaire, ladite première phase.

La centrifugation de l'étape (3°) est une centrifugation supérieure à 500g et se situant dans l'intervalle de 1000 g à 5000 g (soit approximativement, selon les dispositifs de centrifugation usuels, à une vitesse angulaire de 2200 à 4500 tours/minute). Cette centrifugation a une durée d'au moins 5 minutes et de préférence une durée de 10 à 30 minutes. De façon avantageuse, la centrifugation selon l'invention sera réalisée à 2000-4000g pendant 15 minutes.

Ladite centrifugation selon l'invention est en général effectuée à RT. On peut, si cela est requis, réaliser ladite centrifugation à une température comprise entre RT et 45°C (de préférence entre RT et 37°C), c'est-à-dire à une température analogue à celle de la culture prévue à l'étape (4°) pour la pousse des microorganismes pouvant être présents dans ledit échantillon liquide, mais l'utilisation d'une température supérieure à RT n'est pas essentielle pendant la centrifugation.

Quand la phase de protection est présente dans le système gélifié de l'invention, le liquide dudit échantillon liquide migre à travers ladite phase de protection au cours de la centrifugation ; après ladite centrifugation ledit liquide dépourvu des constituants solides non-dissous qui ont également migré se trouve situé entre ladite phase de protection et la phase d'absorption (si celle-ci est présente) ou la phase de révélation (en l'absence de la phase d'absorption dans ledit système gélifié).

La révélation prévue à l'étape (4°) intervient pendant la centrifugation, d'une part, et le cas échéant après culture des microorganismes à une température comprise entre RT et 45°C, d'autre part. La durée de la culture requise pour la pousse des microorganismes est en général fonction de la concentration initiale desdits microorganismes (bactéries et/ou levures) dans ledit échantillon liquide.

En pratique, pour un échantillon liquide ayant une concentration initiale en bactéries inférieure au seuil de détection (i.e. $10^3$ germes/ml), il faut prévoir une durée de culture d'au plus 24h (de préférence une durée de 1-2h), sauf pour les souches de ***Staphylococcus aureus*** qui ont une multiplication relativement lente (pour lesdites souches de ***Staphylococcus*** il faut compter environ 24 h de culture).

Pour un échantillon liquide ayant une concentration initiale en levures inférieure au seuil de détection (i.e. $10^3$ germes/ml), il faut prévoir une durée d'au plus 36 h (de préférence une durée de 24-30 h). Pour favoriser la détection desdites levures, il est recommandé d'incorporer un antibactérien tel que décrit dans le document WO-A-90/03726 précité, notamment la gentamycine, dans la phase d'absorption et/ou la phase de révélation, afin d'inhiber la croissance des bactéries ou de détruire lesdites bactéries.

Le milieu de culture utilisé pour gonfler la phase de révélation comporte une source de carbone, une source d'azote et des oligoéléments, par exemple une peptone (5 à 20 g/l), un sucre (1 à 20 g/l, de préférence 5 g/l), un extrait de levure (1 à 5 g/l) et des vitamines (10 à 20 ml) avec un tampon pour stabiliser le pH à une valeur se situant entre 6,0 et 8,0. Pour les bactéries, ce milieu est constitué par exemple d'un bouillon coeur-cervelle additionné d'un sucre. Pour les levures ce milieu peut être soit identique à celui des bactéries soit différent, par exemple : un milieu YNB + glucose, un milieu Sabouraud, un milieu YNB+maltose (qui sont décrits dans WO-A-90/03726 précité).

Pour les bactéries Gram-négatif, on utilisera avantageusement comme milieu de culture le bouillon coeur-cervelle + sucre contenant en tant que réactif préféré induisant une variation de mesure optique détectable :

- la résazurine, à la concentration finale de 0,05 g/ml (0,2 mM),
- le rouge de phénol, à la concentration finale d'environ 0,065 g/ml (0,17 mM),
- le substrat chromogène H-L-Ala-pNA, à la concentration finale d'environ 0,50 g/l (2 mM), ou
- le substrat fluorogène H-L-Ala-NA (environ 2 mM),

ce milieu étant tamponné à pH 6,5-8,0 au moyen d'un tampon phosphate ou Tris (0,1 M), et pouvant contenir le cas échéant :

- un activateur appartenant à l'ensemble des cations divalents, notamment $Mg^{2+}$, $Ca^{2+}$ ou $Mn^{2+}$ (par exemple une solution de $MnCl_2$ ou $MnSO_4$ à 30 mg/l environ),
- un réactif de diazotation tel que le p-diméthylaminocinnamaldéhyde ou le Fast Blue BB, et/ou
- un ou plusieurs inhibiteurs des réactions parasites liées notamment à la présence d'enzymes dans ledit échantillon liquide à tester (par exemple dans le cas du screening des urines : l'aprotinine pour inhiber l'urokinase urinaire et l'EDTA pour inhiber les phosphatases).

Le ou les inhibiteurs peuvent en pratique être disposés dans la phase de révélation et/ou la phase d'absorption.

Pour les bactéries Gram-positif, on utilisera avantageusement le milieu décrit ci-dessus relativement aux bactéries Gram-négatif avec comme réactif préféré induisant une variation de mesure optique détectable :

- la résazurine à la concentration finale d'environ 0,05 g/ml,
- le substrat chromogène H-L-Leu-pNA à la concentration finale d'environ 2 mM, ou
- le substrat fluorogène H-L-Leu-NA à la concentration finale de 2 mM,

le pH étant tamponné à 6,0-8,0, ledit milieu pouvant comporter le cas échéant un activateur, un réactif de diazotation et/ou un ou plusieurs inhibiteurs comme indiqué ci-dessus.

Pour les levures, on utilisera avantageusement un milieu YNB+sucre tamponné à pH 6,5-8,0, avec comme réactif préféré induisant une variation de mesure optique détectable, le substrat chromogène H-L-Pro-pNA ou un substrat équivalent, un inhibiteur essentiel étant un antibiotique tel que la gentamycine, la streptomycine ou le chloramphénicol pour inhiber la croissance des bactéries. Ledit inhibiteur essentiel peut également être présent dans la phase d'absorption.

D'autres avantages et caractéristiques de l'invention seront mieux compris à la lecture de la description qui va suivre des dessins et d'exemples de réalisation. Bien entendu l'ensemble de ces éléments n'est nullement limitatif mais est donné à titre d'illustration.

Le système gélifié 10 selon l'invention est logé dans un tube de centrifugation à fond conique 6a, à fond sphérique 6b ou à fond pourvu d'un capillaire 6c. Ledit système gélifié 10 comprend au moins une couche, à savoir la phase de révélation 1 qui est un gel préalablement gonflé au moyen d'un milieu aqueux de culture de microorganismes, et le cas échéant une ou plusieurs autres couches, à savoir la phase d'absorption 2 qui est soit un gel préalablement gonflé avec de l'eau et pouvant contenir un ou plusieurs inhibiteurs (tels que l'aprotinine et l'EDTA), soit une couche particulaire (silice ou kaolin) micronisée à une granulométrie inférieure à 0,1 μm, la phase barrière 3 qui est un gel préalablement gonflé avec de l'eau ou une couche de paraffine solide dans les conditions de centrifugation et de culture, et/ou la phase de protection 4 qui est une couche de paraffine liquide dans lesdites conditions.

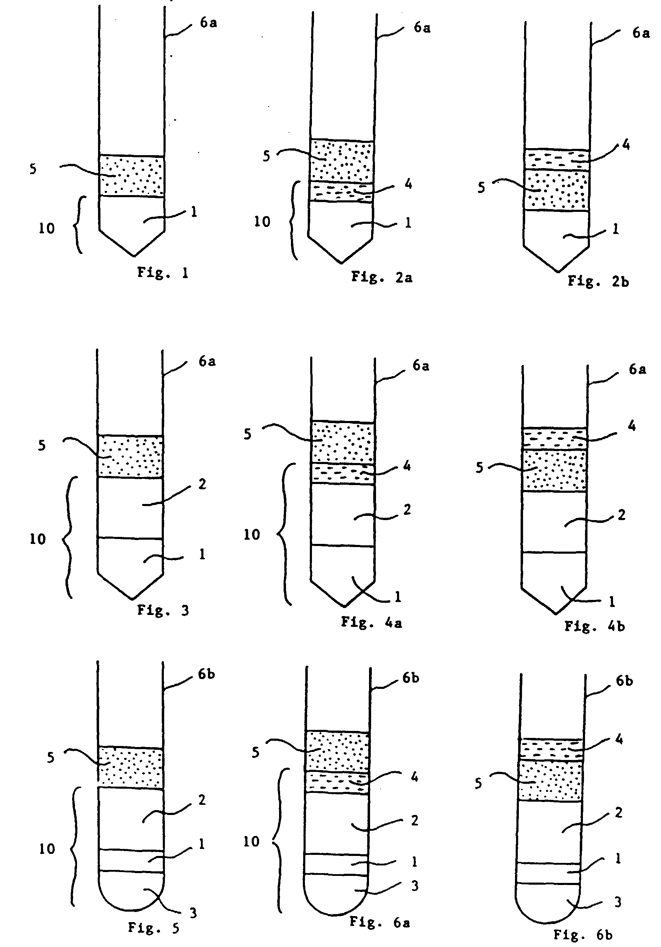

La figure 1 a trait à un système gélifié 10 selon l'invention comportant une seule couche, la phase de révélation 1, logée dans un tube de centrifugation à fond conique 6a. L'échantillon liquide 5 est versé au-dessus de ladite phase de révélation 1. Par centrifugation les particules non dissoutes dans ledit échantillon liquide migrent dans l'épaisseur de la phase de révélation, en revanche le liquide dudit échantillon liquide 5 et les ingrédients dissous ne traversent pas la phase de révélation. Après ladite centrifugation, les particules les plus grosses qui sont les plus lourdes se trouvent réparties au fond du tube 6a en fonction de leurs tailles, au fond les cellules et, au-dessus desdites cellules, les microorganismes qui sont situés au voisinage de la partie inférieure de la phase de révélation 1.

Le système gélifié selon la figure 1 donne de bons résultats, cependant il n'est pas totalement satisfaisant en ce sens que le gel de la phase de révélation 1 (i) est au contact dudit échantillon liquide 5 pendant la centrifugation et la culture, et (ii) peut par suite être dilué par ledit échantillon liquide 5.

Les figures 2a et 2b ont trait au même système gélifié 10 avant (fig. 2a) et après (fig. 2b) centrifugation. Par rapport au système gélifié de la figure 1, le système gélifié des figures 2a et 2b comporte en outre une couche d'huile (paraffine liquide) constituant la phase de protection. On verse dans le tube 6a la phase de révélation 1 puis la phase de protection 4, procède à une centrifugation (2000-3000 g pendant 10-20 minutes) pour tasser ledit système gélifié 10. On verse ensuite dans le tube 6a l'échantillon liquide 5 à tester. Ledit échantillon liquide 5 se répartit au-dessus de la phase de protection 4 (voir figure 2a) avant centrifugation.

Lors de la centrifugation ledit échantillon liquide 5 migre à travers la phase de protection 4 avec les ingrédients solubles qu'il contient. Ses particules solides migrent à travers la phase de révélation 1, les plus lourdes desdites particules se répartissent au fond du tube comme indiqué ci-dessus pour la figure 1. Après centrifugation, le liquide de l'échantillon liquide 5 est situé entre la phase de protection 4 et la phase de révélation 1 (voir figure 2b).

Le système gélifié des figures 2a et 2b est certes meilleur que celui de la figure 1, il présente néanmoins l'inconvénient d'un contact de l'échantillon liquide 5 avec la phase de révélation 1 après centrifugation pendant l'étape dite de culture.

La figure 3 a trait à un système gélifié 10 selon l'invention comprenant deux couches : la phase de révélation 1 et la phase d'absorption 2. On verse dans le tube de centrifugation à fond conique 6a la phase de révélation 1 puis la phase d'absorption 2, centrifuge (1000-3000 g pendant 10-20 minutes) pour tasser ledit système gélifié 10. On verse ensuite dans le tube 6a l'échantillon liquide 5 à tester. Ledit échantillon liquide 5 se répartit au-dessus de la phase d'absorption.

Lors de la centrifugation de l'étape (3°), les particules solides contenues dans l'échantillon liquide 5 migrent en fonction de leurs tailles à travers la phase d'absorption 2 et/ou la phase de révélation 1, en revanche le liquide dudit échantillon liquide 5 et les ingrédients qui y sont dissous ne traversent pas la phase d'absorption 2.

Après la centrifugation de l'étape (3°), on retrouve au fond du tube 6a les particules les plus grosses et lourdes dudit échantillon liquide 5 selon la répartition donnée ci-dessus pour les figures 1 et 2a-2b.

Le fait que la phase de révélation 1 soit isolée du liquide dudit échantillon liquide 5 par la phase d'absorption 2, après la centrifugation de l'étape (3°), d'une part, et que la phase d'absorption 2 absorbe les particules de faible taille, d'autre part, constitue un avantage.

Les figures 4a [avant centrifugation] et 4b [après centrifugation selon l'étape (3°)] ont trait à un système gélifié 10 selon l'invention analogue à celui de la figure 3 mais comportant en plus, dans un tube de centrifugation à fond conique 6a, une couche d'huile (huile de paraffine), à savoir la phase de protection 4. On verse dans le tube 6a la phase de révélation 1, la phase d'absorption 2 puis la phase de protection 4, procède à une centrifugation (1000-3000 g pendant 10-20 minutes) pour tasser ledit système gélifié 10. On verse ensuite dans le tube 6a l'échantillon liquide 5 à tester. Ledit échantillon liquide 5 se répartit au-dessus de la phase de protection 4 (voir figure 4a).

Lors de la centrifugation de l'étape (3°) l'échantillon liquide 5 migre à travers la phase de protection 4, et les particules solides qu'il contient migrent également en fonction de leur taille à travers la phase d'absorption 2 et/ou la phase de révélation 1, en revanche le liquide dudit échantillon liquide 5 et les ingrédients qui y sont dissous ne traversent pas la phase d'absorption 2. Après centrifugation, les particules les plus grosses et les plus lourdes se trouvent réparties au fond du tube 6a comme indiqué plus haut.

La figure 5 a trait à un système gélifié 10 selon l'invention comportant trois couches, à savoir de bas en haut : la phase barrière 3, la phase de révélation 1 et la phase d'absorption 2, la couche 1 étant un gel, la couche 2 étant soit un gel soit une phase particulaire, et la couche 3 étant soit un gel soit une paraffine solide.

Dans un tube à fond sphérique 6b, on verse successivement la phase barrière 3 (préalablement fondue, si elle est constituée par une paraffine solide à une température de RT à 50-55°C, puis refroidie), la phase de révélation 1 puis la phase d'absorption 2. On procède à une centrifugation (1000-3000 g pendant 10-20 minutes) pour tasser ledit système gélifié 10. On verse ensuite dans le tube 6b l'échantillon liquide 5 à tester. Ledit échantillon liquide 5 se répartit au-dessus de la phase d'absorption 2 avant la centrifugation de l'étape (3°).

Lors de la centrifugation de l'étape (3°) le liquide dudit échantillon liquide 5 et les constituants dissous qu'il contient ne migrent pas à travers la phase d'absorption 2 ; seules migrent à travers le système gélifié 10 constitué par l'ensemble phase d'absorption/phase de révélation/phase barrière, en fonction de leur taille, les particules solides dudit échantillon liquide 5. Quand la phase barrière 3 est une paraffine solide toutes les particules grosses et lourdes ne traversent pas son épaisseur mais se répartissent en fonction de leur taille au niveau de l'interface phase de révélation/ phase barrière.

La configuration des couches 2, 1 et 3 selon la figure 5 (il en est de même pour le système gélifié des figures 6a et 6b) offre l'avantage de pouvoir diminuer l'épaisseur de la phase de révélation 1 prise en sandwich entre la phase d'absorption 2 et la phase barrière 3 et d'améliorer ainsi la lecture de la variation de OM soit à l'oeil nu (changement de couleur) soit au moyen d'un colorimètre.

Les figures 6a [avant la centrifugation de l'étape (3°)] et 6b [après la centrifugation de l'étape (3°)] ont trait à un système gélifié 10 selon l'invention, qui est analogue à celui de la figure 5, mais comportant en outre une couche d'huile 4 (huile de paraffine) constituant la phase de protection. La répartition des particules solides de l'échantillon liquide 5 s'effectue comme indiqué pour la figure 5, la phase de protection 4 permettant la migration dudit échantillon liquide 5 lors de la centrifugation de l'étape (3°).

Les substances polymériques qui interviennent à l'état gonflé dans les gels (de haut en bas) de la phase d'absorption 2, de la phase de révélation 1 et de la phase barrière 3, sont avantageusement des particules de dextrane. Conviennent en particulier les produits de la gamme Sephadex® G commercialisés par la société dite PHARMACIA FINE CHEMICALS (Uppsala, Suède), à savoir :

- pour la phase d'absorption 2, le produit G 25C,
- pour la phase de révélation 1, les produits G 25 à G 100 de granulométrie dite grossière à moyenne, tels que G 25M, G 50C, G 50M, G 75 et G 100 (le produit préféré étant G 100),
- pour la phase barrière 3, les produits G 150 à G 200 de granulométrie dite moyenne à superfine, tels que les G 150, G 150S, G 200 et G 200S (les produits préférés étant G 200 et surtout G 200S).

Les caractéristiques de ces produits du type dextrane sont consignées dans le tableau I ci-après.

TABLEAU I

| Phase | Gel | (A) | Domaine de fractionnement (MW) | |
|---|---|---|---|---|
| | | | (B) | (C) |
| Absorption | G 25C | 100-300 | 1000-5000 | 100-5000 |

TABLEAU I   (suite)

| Phase | Gel | (A) | Domaine de fractionnement (MW) | |
|---|---|---|---|---|
| | | | (B) | (C) |
| Révélation | G 25M | 50-150 | 1500-30000 | 500-10000 |
| | G 50C | 100-300 | 1500-30000 | 500-1000 |
| | G 50M | 50-150 | 1500-30000 | 500-10000 |
| | G 75 | 40-120 | 3000-80000 | 1000-50000 |
| | G100 | 40-120 | 4000-150000 | 1000-100000 |
| Barrière | G 150 | 40-120 | 5000-300000 | 1000-150000 |
| | G 150S | 10-40 | 5000-150000 | 1000-150000 |
| | G 200 | 40-120 | 5000-600000 | 1000-200000 |
| | G 200S | 10-40 | 5000-250000 | 1000-200000 |

Notes :
(A) : diamètre en μm des particules à l'état sec
(B) : peptides et protéines globulaires
(C) : dextranes
G 25C : "Sephadex® G 25 Coarse"
G 25M : "Sephadex® G 25 Medium"
G 50C : "Sephadex® G 50 Coarse"
G 50M : "Sephadex® G 50 Medium"
G 75 : "Sephadex® G 75"
G 100 : "Sephadex® G 100"
G 150 : "Sephadex® G 150"
G 150S : "Sephadex® G 150 Superfine"
G 200 : "Sephadex® G 200"
G 200S : "Sephadex® G 200 Superfine"

Dans les tableaux II et III ci-après, on a donné pour les gels préférés, les caractéristiques relatives à la granulométrie à l'état gonflé et à la concentration, qui sont importantes selon l'invention.

TABLEAU II

| Phase | Gel | Diamètre des grains | (μm) à l'état gonflé |
|---|---|---|---|
| | | (A) | (B) |
| Absorption | G 25C | 160-530 | 172-516 |
| Révélation | G 100 | 90-320 | 103-311 |
| Barrière | G 200S | 30-130 | 32-129 |

Notes :
(A) : domaine de l'invention
(B) : domaine préféré

TABLEAU III

| Phase | Gel | Concentration (g/ml) | | |
|---|---|---|---|---|
| | | (A) | (B) | (C) |
| Absorption | G 25C | 0,2 | 0,35 | 1 |
| Révélation | G 100 | 0,05 | 0,1 | 0,2 |

TABLEAU III   (suite)

| Phase | Gel | Concentration (g/ml) | | |
|---|---|---|---|---|
| | | (A) | (B) | (C) |
| Barrière | G 200S | 0,04 | 0,14 | 0,2 |

Notes :
(A) : concentration minimale selon l'invention
(B) : concentration préférée selon l'invention
(C) : concentration maximale selon l'invention

En se référant aux concentrations minimales et maximales des gels fournis dans le tableau m, il convient de remarquer que :

(1) si le gel en contact avec le liquide de l'échantillon liquide 5 (i.e. couche 1 de la figure 1 ou couche 2 des figures 3 et 5) est plus concentré, il y a un risque de passage dudit échantillon liquide 5 à travers ledit gel pendant la centrifugation ;
(2) si tous les gels sont plus concentrés, la détection de la présence ou de l'absence de microorganismes est gravement perturbée ;
(3) si l'un au moins des gels est plus dilué, on peut avoir des échanges de liquide entre l'échantillon liquide et le gel sous-jacent, d'une part, ou entre deux gels adjacents, d'autre part, ce qui fausse ladite détection.

On peut supprimer la phase barrière 3, comme cela est illustré par les figures 1, 2a-2b, 3 et 4a-4b, lorsque les grosses particules (érythrocytes, leucocytes, autres cellules) n'ont pas d'activité sur le réactif induisant une variation de mesure optique détectable. Cela est notamment le cas quand ledit réactif est la résazurine.

On peut supprimer la phase d'absorption 2 et la phase barrière 3 comme cela est illustré par les figures 1 et 2a-2b, quand aucune substance (dissoute ou particulaire) présente dans l'échantillon liquide 5 n'a d'activité sur ledit réactif induisant une variation de mesure optique détectable ou lorsque toute interférence possible peut être inhibée pendant la révélation de l'étape (4°).

Si la concentration des microorganismes, qui sont éventuellement présents dans l'échantillon liquide 5 à tester et qui ont migré lors de la centrifugation de l'étape (3°) pour se trouver dans la phase de révélation 1 (soit dans l'épaisseur de ladite phase 1, soit au-dessus de l'interface phase 1/phase 3 ou phase 1/paroi inférieure du tube 6a), est supérieure au seuil de détection du réactif de la phase de révélation 1 induisant une variation de mesure optique détectable il n'y a pas lieu de procéder à la multiplication des microorganismes par culture *in situ* à l'étape (4°).

En revanche, si après ladite centrifugation la concentration des microorganismes est inférieure au seuil de détection, il est requis d'entreprendre ladite multiplication par culture *in situ* dans le tube de centrifugation au niveau de ladite phase de révélation 1, de préférence à une température de 37°C.

Le tube de centrifugation 6a, 6b ou 6c est réalisé en un matériau transparent (plastique ou verre). De préférence, le tube de centrifugation utile selon l'invention sera réalisé en plastique et fabriqué par moulage. En variante, ledit tube peut être transparent sur au moins une partie de sa surface périphérique en regard de la zone garnie avec la phase de révélation 1.

Selon l'invention, le seuil de détection des microorganismes, rapporté à la concentration initiale desdits microorganismes dans ledit échantillon liquide 5, est de $10^3$ germes/ml, c'est-à-dire nettement inférieur au seuil de $10^5$ germes/ml des techniques antérieures illustrées notamment par les tests Bac-T-Screen® et FiltraCheck®-UTI précités.

Cette amélioration est due au fait que, selon l'invention, on ($\alpha$) concentre les microorganismes de l'échantillon liquide 5, qui ont migré lors de la centrifugation, dans la phase de révélation 1, puis ($\beta$) le cas échéant, les fait multiplier dans le milieu nutritif.

Pour concentrer les microorganismes dans la phase de révélation, il est recommandé selon l'invention de faire appel à une phase de révélation 1 ayant un volume et une épaisseur les plus faibles possibles. Par suite, la forme du tube de centrifugation sera choisie de telle façon que le facteur de concentration des microorganismes, qui est directement proportionnel au rapport volume de l'échantillon à tester/volume de la phase de révélation, soit maximal. Le volume de la phase de révélation est déterminé par la formule $V = \pi R^2 h$ (où V est exprimé en ml, R le rayon et h la hauteur sont exprimés en cm). Plus le diamètre du tube est faible, plus le facteur de concentration augmente. A titre d'exemple, le facteur de concentration peut être un multiple de $10^3$ quand le rayon du tube est divisé par 10.

Le capillaire du tube 6c permet une bonne concentration des microorganismes dans la phase de révélation.

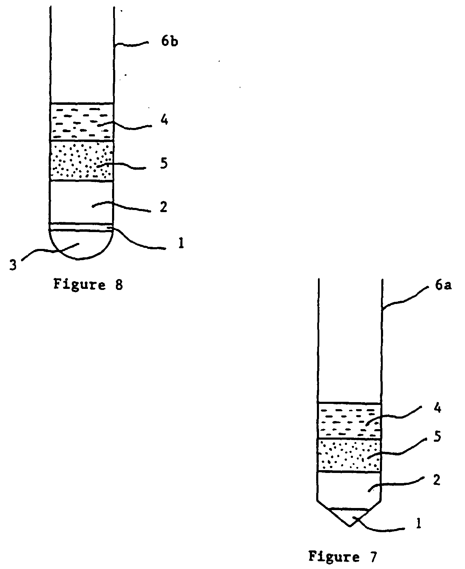

Les figures 7 et 8 montrent des systèmes gélifiés préférés selon l'invention, tels qu'ils se présentent après centrifugation. Le tube de la figure 7, qui a un contenu similaire à celui de la figure 4b, comporte une phase de révélation 1 de volume plus faible que ceux de la phase 2 et de l'échantillon 5, qui est logée au fond dudit tube de centrifugation 6a dans la portion inférieure de la partie conique.

Le tube 6b de la figure 8, qui a un contenu similaire à celui de la figure 6b, présente un fond sphérique (qui peut avoir un diamètre plus petit que celui du reste du tube) où est logé la phase barrière 3 et la phase de révélation 1. Ce fond est tel que la phase de révélation 1 prise en sandwich entre la phase barrière 3 inférieure et la phase d'absorption 2 supérieure ait un volume et une épaisseur les plus faibles possibles.

A titre d'exemple, si on fait appel à un tube en plastique transparent, selon la figure 8, ayant un volume de 12 ml, un diamètre intérieur de 1,35 cm et dans lequel on introduit un volume de 6 ml d'échantillon d'urine à tester, pour obtenir la phase de révélation 1 sous la forme d'un anneau de 0,2 cm de hauteur, il faut que le volume de ladite phase 1 à utiliser ait pour valeur de $V = \pi.(1,35/2)^2.0,2$ i.e., environ 0,3 ml. Le facteur de concentration (F) des microorganismes de l'urine est alors de 6/0,3 = 20 pour qu'un anneau coloré soit visible. Comme la réaction positive débute par un point représentant moins du dixième du diamètre du tube, le volume réactionnel utile de la phase de révélation 1 devient alors de 0,003 ml, ce qui conduit à un facteur de concentration de 6/0,003 = 2000, soit une augmentation du seuil de sensibilité du facteur de concentration d'un multiple de $10^3$ visé ci-dessus. En variante, lorsque la section de la partie inférieure du tube est diminuée d'un facteur 10 (i.e. divisée par 10 ; soit 0,135 cm de diamètre), V devient 0,3 x $10^{-2}$ ml, soit une augmentation de $10^2$ du facteur F pour un anneau et une augmentation de $10^3$ dudit facteur F pour un point.

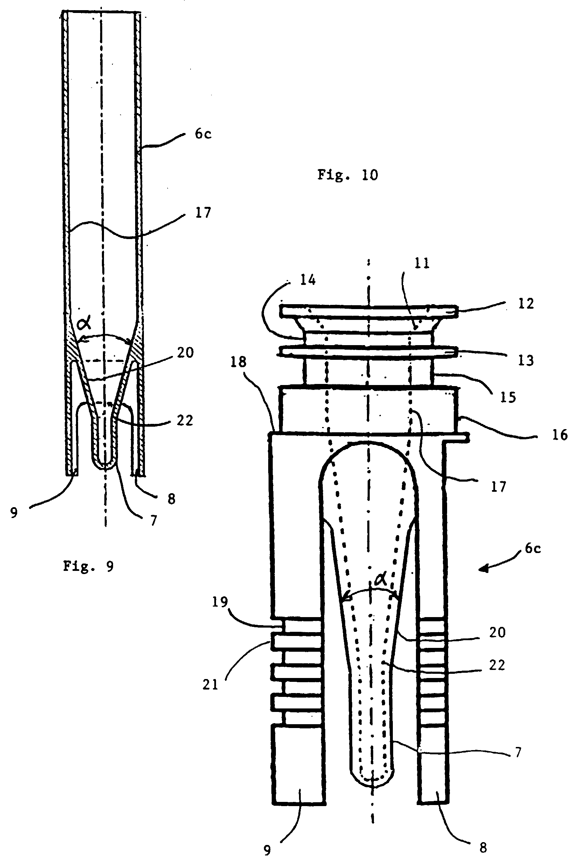

Les figures 9 et 10 présentent chacune un tube de centrifugation 6c qui est en matière plastique transparente (par exemple en polystyrène ou polycarbonate) et qui est utile selon l'invention. Le tube 6c offre l'avantage de pouvoir procéder à une numération des microorganismes présents dans la phase de révélation logée dans le capillaire.

Selon la figure 9, le tube 6c est pourvu à son extrémité inférieure d'un capillaire 7 situé le long de l'axe dudit tube et destiné à recevoir la phase de révélation (référencée 1 ci-dessus). La partie cylindrique du tube 6c est en relation avec l'ouverture du capillaire 7 par l'intermédiaire d'une portion tronconique 20. La phase de révélation est logée dans le tube capillaire jusqu'à la ligne 22 qui correspond à l'intersection du tronc de cône 20 avec l'ouverture du capillaire 7. Au-dessus de la ligne 22 est logée, le cas échéant, la phase de protection (référencée 4 ci-dessus) et/ou la phase d'absorption (référencée 2 ci-dessus). La paroi cylindrique extérieure du tube 6c se prolonge vers le bas en regard du capillaire par deux pattes (ou prolongements) 8 et 9 qui sont sensiblement diamétralement opposés et assurent la stabilité du tube quand il est disposé debout. Ces pattes 8 et 9 constituent un socle ; entre elles sont ménagées deux fenêtres permettant d'observer à l'oeil nu ou au moyen d'un dispositif optique (colorimètre, spectromètre, etc.) le contenu du capillaire 7 avant centrifugation, puis après centrifugation ou culture en vue de procéder à la numération des bactéries ou des levures présentes.

Le tube 6c de la figure 10 est réalisé de façon à procéder à la numération des bactéries ou des levures présentes comme indiqué ci-dessus pour le tube de la figure 9, d'une part, et à recueillir lesdites bactéries ou levures présentes afin de les identifier, d'autre part. Ce tube comporte un capillaire 7, une paroi tronconique 20 et une paroi cylindrique interne 17, l'intersection de la surface 20 avec l'ouverture du capillaire 7 constituant la ligne 22. L'ouverture de la portion cylindrique 17 vers l'extérieur est soit parallèle à l'axe du tube 6c, soit en forme d'entonnoir comme représenté en 11.

La surface extérieure du tube 6c de la figure 10 comporte au voisinage de l'extrémité supérieure une ou plusieurs gorges 14 et/ou 15 où peut être logé un joint torique (notamment en caoutchouc) non représenté ici, l'une de ces gorges 14 est disposée entre deux collerettes ou lèvres 12 et 13, une autre de ces gorges 15 est disposée entre la collerette 13 et la surface extérieure 16 du tube. Une butée annulaire 18 est prévue sur ladite surface extérieure 16. L'ensemble collerette 12 et/ou 13 + surface extérieure 16 est destiné à être obturé par un bouchon cylindrique notamment en matière plastique recouvrant la partie supérieure externe du tube 6c jusqu'à la butée 18. Les pattes ou prolongements 8 et 9 servent de socle pour maintenir debout le tube de centrifugation 6c comme indiqué ci-dessus pour le tube de la figure 9. L'une de ces pattes 8 peut présenter une portion de surface dirigée vers l'extérieur qui est plane, afin de recevoir le cas échéant, des indications manuscrites ou collées. Les pattes 8 et 9 peuvent également comporter sur leur surface dirigée vers l'extérieur un ensemble de plusieurs gorges 19 et de lèvres 21 alternées, de façon à pouvoir, si cela est nécessaire, loger le tube 6c de la figure 10 (qui peut avoir une hauteur très petite notamment 30 mm) dans l'ouverture supérieure d'un autre tube de centrifugation (plus grand) convenant au dispositif de centrifugation utilisé.

L'observation et la numération des bactéries ou levures présentes dans la phase de révélation logée dans le capillaire 7 jusqu'à la hauteur de la ligne 22 sont effectuées comme indiqué plus haut pour le tube de la figure 9 au moyen des fenêtres ménagées entre les pattes 8 et 9.

Pour recueillir les microorganismes présents dans la phase de révélation logée dans le capillaire 7 du tube de la figure 10, (1°) on retourne ledit tube pour jeter le surnageant, ladite phase de révélation restant dans le capillaire, (2°) on place un joint torique dans la gorge 14, (3°) on insère l'ensemble collerette 12 + joint logé dans la gorge 14 + collerette 13 + surface 16 à l'intérieur de l'ouverture d'un second tube de centrifugation contenant le milieu liquide de récupération, jusqu'à ce que le second tube de centrifugation vienne en contact avec la butée 18, le tube 6c étant maintenu retourné (c'est-à-dire capillaire 7 dirigé vers le haut), (4°) on agite l'ensemble des deux tubes de centrifugation ainsi raccordés par leurs ouvertures, puis (5°) on centrifuge, à 1000-2000 g, pendant 2-5 minutes, pour faire passer la phase de révélation, initialement contenu dans le capillaire 7, dans le liquide de récupération qui se trouve au fond du second tube.

Cette façon de procéder permet la récupération aisée des microorganismes présents sans contamination et sans perte notable. Cette technique est bien plus efficace que celle qui consiste à procéder au prélèvement de la phase de révélation logée dans le capillaire 7 au moyen d'une aspiration avec une seringue ; en effet les particules polymériques gonflées du gel constituant la phase de révélation ne passent pas facilement à travers l'aiguille de la seringue du fait de leurs dimensions.

En variante, on peut recueillir la majeure partie de la phase de révélation contenant des microorganismes en utilisant le tube de la figure 9. Dans ce but, (1a) on jette le surnageant situé au-dessus de la ligne 22, (2a) on ajoute une solution aqueuse de lavage, obture le tube 6c et agite, (3a) on centrifuge à 1000-2000 g pendant 2-5 minutes, (4a) on reproduit les opérations (2a) et (3a) jusqu'à ce que le surnageant soit incolore, (5a) on jette le surnageant résultant, ajoute une solution aqueuse de récupération et agite pour recueillir le milieu liquide résultant contenant la majeure partie de la phase de révélation et par suite des microorganismes présents.

D'un point de vue pratique, pour que la phase de révélation reste dans le capillaire lorsque le tube 6c est retourné avant agitation puis centrifugation, il est important que le diamètre interne du capillaire soit inférieur ou égal à 2,5 mm. Un diamètre interne convenable se situe entre 1,2 et 2,3 mm. On a obtenu de très bons résultats avec des diamètres intérieurs de 2,0 et 2,2 mm.

Pour améliorer la sensibilité de la détection des microorganismes et obtenir une bonne récupération de la phase de révélation, il est recommandé de satisfaire au moins une des conditions suivantes et de préférence l'ensemble desdites conditions :

A- le diamètre interne du capillaire est inférieur à 2,5 mm,

B- l'angle $\alpha$ de la portion tronconique 20 est inférieur ou égal à 30° (i.e. $\leq \pi/6$), c'est-à-dire que la génératrice de cette portion tronconique est faiblement inclinée par rapport à l'axe vertical du tube (angle d'inclinaison inférieur ou égale à $\alpha/2$),

C- le volume du capillaire est inférieur ou égal à 0,6 ml et de préférence inférieur ou égale à 0,1 ml.

Si la condition B ou la condition C n'est pas satisfaite, il y a un risque de dépôt de traces de phase de révélation sur la paroi interne du tube et par suite de microorganismes, surtout au-dessus du voisinage de l'intersection 22.

## Exemple 1

Dans un tube de centrifugation en plastique transparent, d'un volume de 5 ml et d'un diamètre intérieur de 1,10 cm on introduit successivement :

- 0,5 ml d'un gel G 200S/eau distillée, dans lequel G 200S est à une concentration en poids sec de 0,2 g/ml et a une granulométrie à l'état gonflé de 40-100 μm, pour former la phase barrière ;
- 0,25 ml d'un gel G 100/milieu de culture aqueux, dans lequel G 100 est à une concentration en poids sec de 0,2 g/ml et a une granulométrie à l'état gonflé de 120-300 μm, ledit milieu de culture aqueux ayant la composition suivante :

| bouillon de coeur-cervelle | 37 g/l |
|---|---|
| glucose | 5 g/l |
| agarose (type IX) | 0,33 g/l |
| pH | 7,4 ± 0,1 |

et contenant 0,05 g/ml de résazurine, pour former la phase de révélation ; et,
- 0,5 ml d'un gel G 25C/eau distillée, dans lequel G 25C est à une concentration en poids sec de 1 g/ml et a une granulométrie à l'état gonflé de 40-110 μm, pour former la phase d'absorption.

On procède à une centrifugation (1000-3000 g pendant 10-20 minutes à RT) pour tasser le système gélifié résultant. En variante, ladite centrifugation de tassage peut être effectuée après l'introduction de chacune desdites phases avant d'introduire la phase suivante.

On dispose d'un système gélifié prêt à l'emploi.

## Exemple 2

On introduit, au-dessus du système gélifié préparé selon l'exemple 1 ci-dessus, 1 ml d'huile de paraffine en tant que phase de protection.

On obtient ainsi un dispositif pour détecter la présence ou l'absence de microorganismes dans un échantillon d'un matériau biologique, ledit dispositif étant stable au stockage.

***Exemple 3***

Dans un tube de centrifugation en plastique transparent, d'un volume de 5 ml et d'un diamètre intérieur de 1,10 cm, on introduit successivement :

- 0,5 ml de paraffine fondant à une température supérieure ou égale à 50°C (l'introduction de cette paraffine est effectuée à l'état fondu), pour former (après refroidissement jusqu'à RT) la phase barrière ;
- 0,25 ml d'un gel G 100/milieu de culture aqueux, dans lequel G 100 est à une concentration en poids sec de 0,2 g/ml et a une granulométrie à l'état gonflé de 103-311 µm, ledit milieu de culture aqueux ayant la composition suivante :

| bouillon de coeur-cervelle | 37 g/l |
|---|---|
| glucose | 5 g/l |
| agarose (type IX) | 0,33 g/l |
| pH | $7,4 \pm 0,1$ |

et contenant 2 mM de H-L-Ala-NA en tampon phosphate (0,1 M) à pH 8,0, pour former la phase de révélation ; et,
- 0,5 ml d'un gel G 25C/eau distillée dans lequel G 25C est à une concentration en poids sec de 1 g/ml et a une granulométrie à l'état gonflé de 32-129 µm, l'eau distillée utilisée pour élaborer ce gel contenant 0,06 g/ml d'EDTA, pour former la phase d'absorption.

On procède à une centrifugation (1000-3000 g pendant 10-20 minutes à RT) pour tasser le système gélifié résultant. En variante, ladite centrifugation de tassage peut être effectuée après l'introduction de chacune desdites phases avant d'introduire la phase suivante.

On dispose d'un système gélifié prêt à l'emploi.

***Exemple 4***

On introduit, au-dessus du système gélifié préparé selon l'exemple 3 ci-dessus, 0,25 ml d'huile de paraffine en tant que phase de protection.

On obtient ainsi un dispositif pour détecter la présence ou l'absence de microorganismes dans un échantillon d'un matériau biologique, ledit dispositif étant stable au stockage.

***Exemple 5***

On procède comme indiqué à l'exemple 3 avec la différence que le milieu de culture aqueux utilisé pour le gonflement de G100 en vue d'obtenir la phase de révélation contient en outre un réactif de diazotation, le p-diméthylaminocinnamaldéhyde.

***Exemple 6***

Dans un tube de centrifugation en plastique transparent ayant un fond conique, un diamètre intérieur (dans sa portion cylindrique) de 1 cm et un volume de 5 ml, on introduit successivement :

- 0,20 ml d'un gel G 100/milieu de culture aqueux, dans lequel G 100 est à une concentration en poids sec de 0,2 g/ml et a une granulométrie à l'état gonflé de 103-311 µm, ledit milieu de culture aqueux ayant la composition suivante :

| YNB | 6,7 g/l |
|---|---|
| maltose | 20,0 g/l |
| gentamycine | 0,05 g/l |
| cycloheximide | 0,5 g/l |

(suite)

| pH | 6,0 |
|---|---|

et contenant 1 mM de H-L-Pro-pNA (ce milieu de culture contenant un substrat spécifique des souches de **Candida albicans** permettant de distinguer lesdits **Candida albicans** des autres **Candida**), pour former la phase de révélation ; et,

- 0,5 ml de kaolin micronisé et ayant une granulométrie homogène de 0,055-0,060 µm, pour former la phase d'absorption.

On tasse le système gélifié résultant par centrifugation à 2000 g pendant 15 minutes à RT.

On dispose ainsi d'un système gélifié prêt à l'emploi, qui est spécifiquement destiné à l'identification des **Candida albicans** parmi un ensemble de souches non isolées de **Candida**.

**Exemple 7**

On introduit au-dessus du système gélifié préparé selon l'exemple 6, 1 ml d'huile de paraffine, pour former la phase de protection et on centrifuge à 2000 g pendant 15 minutes à RT pour tasser le système gélifié résultant.

**Exemples 8 et 9**

On procède comme indiqué à l'exemple 6 ci-dessus mais en utilisant comme milieu nutritif 0,20 ml du milieu nutritif de l'exemple 1 contenant de la résazurine pour l'obtention de la phase de révélation afin de préparer un système gélifié spécifique de la détection des bactéries (exemple 8). A partir du système gélifié ainsi obtenu, on prépare selon l'exemple 7 un système gélifié prêt à l'emploi qui est pourvu de la phase de protection (exemple 9).

**Exemples 10 et 11**

On procède comme indiqué à l'exemple 6 ci-dessus mais en utilisant comme milieu nutritif 0,20 ml d'un milieu nutritif spécifique des souches d'**Aspergillus** pour l'obtention de la phase de révélation afin de préparer un système gélifié spécifique de la détection de ces levures (exemple 10). A partir du système gélifié ainsi obtenu, on prépare selon l'exemple 7 un système gélifié prêt à l'emploi qui est pourvu de la phase de protection (exemple 11).

**Exemple 12**

On a introduit dans les tubes contenant les systèmes gélifiés préparés selon les exemples 1 et 5, un volume de 1 à 4 ml d'urine stérilisée (où on a introduit différentes concentrations de souches d'**Escherichia coli**) ou d'urine d'un patient atteint d'infection urinaire provoquée par des souches d'**Escherichia coli**. Après 20 minutes de centrifugation à 3000-5000 g et à RT on observe, avec un échantillon d'urine d'infection urinaire contenant des souches d'**Escherichia coli** à la concentration de $10^5$ CFU/ml, un point rose avec la résazurine (i.e. système gélifié préparé selon l'exemple 1) et un point rouge avec le mélange H-L-Ala-NA/réactif de diazotation (i.e. système gélifié préparé selon l'exemple 5). Ces points colorés situés au niveau de la phase de révélation s'étendent et forment chacun un anneau coloré après quelques heures à RT ou 37°C.

Les résultats obtenus avec la résazurine ont été consignés ci-après dans le tableau IV.

TABLEAU IV

| Détection de souches d'Escherichia coli avec la résazurine | | | | | | |
|---|---|---|---|---|---|---|
| Lecture | Bactéries/ml en urine d'infection urinaire | | | | | |
| | $10^8$ | $10^7$ | $10^6$ | $10^5$ | $\leq 10^4$ | $10^3$ |
| à t = 0,5 h | +++ | +++ | +++ | ++ | + | +/- |
| à t = 2 h | +++ | +++ | +++ | +++ | +++ | +++ |

**Exemple 13**

Des essais complémentaires ont été réalisés sur des lots d'urine provenant de 50 personnes (35 personnes étant atteintes d'infection urinaire bactérienne, 15 personnes utilisées pour contrôle ne souffrant pas d'infection urinaire). Les systèmes gélifiés des exemples 1-5 ont été utilisés simultanément pour chaque échantillon d'urine testé 2 ml d'urine ont été introduits dans chacun des tubes de centrifugation.

Après centrifugation à 3000-5000 g pendant 15 minutes à RT puis culture *in situ* à 37°C pendant 24 h, on a observé avec ces échantillons d'urine une amélioration des Sen, Spe et NPV (pour de faibles concentrations bactériennes) avec les systèmes gélifiés selon l'invention par rapport aux tests Bac-T-Screen® et FiltraCheck®-UTI précités. Une partie des résultats obtenus est consignée ci-après pour une concentration dans les échantillons d'urine de $10^3$ bactéries/ml :

$$Sen = 95\text{-}98\ \%$$

$$Spe = 93\text{-}97\ \%$$

$$NPV = 91\text{-}94\ \%$$

## Exemple 14

Le système gélifié de l'exemple 2 a été utilisé pour apprécier la présence ou l'absence de bactéries dans 8 blocs opératoires. Les tubes (d'un volume de 15 ml), garnis dudit système gélifié et pourvus ou non d'une collerette du type entonnoir au niveau de leur orifice supérieur, ont été disposés pendant 24 h dans des blocs opératoires au voisinage des arrivées de gaz, à une hauteur de 1,50 m et à une distance de 1 m du mur le plus proche. Avec une pipette, on a fait couler de l'eau distillée pour rincer la partie exposée de chaque tube et de chaque collerette présente. Après centrifugation (30 minutes à 3000 g) et culture *in situ* à 37°C pendant 24 h, on a pu détecter dans l'un des blocs opératoires la présence de bactéries dans l'atmosphère ambiante (principalement des souches de ***Staphylococcus***). Le bloc opératoire contaminé a été stérilisé.

## Exemple 15

On a découpé puis finement dilacéré les filtres (membranes cellulosiques) des conduits d'aération de 6 blocs opératoires. Le matériau ainsi obtenu a été trituré avec de l'eau distillée ; le surnageant résultant a été introduit à raison de 1 à 2 ml dans des tubes obtenus selon le procédé des exemples 2 et 11. Après centrifugation à 3000-5000 g pendant 15 minutes à RT puis culture in situ à 37°C pendant 24 h, on a pu détecter dans l'un des six blocs opératoires la présence de bactéries (principalement des souches de ***Pseudomonas***) et dans un autre desdits blocs opératoires la présence de levures (dans le cas d'espèce des souches d'***Aspergillus***). Les deux blocs opératoires contaminés ont été stérilisés.

## Exemple 16

Du lait "clarifié" (i.e. délipidé) et stérilisé a été contaminé avec des souches de ***Candida*** (à une concentration supérieure ou égale à 105 germes/ml) et réparti à raison de 2,5 ml dans des lots de tubes dc centrifugation (5 tubes par lot) préparés selon le procédé de l'exemple 7 :

- le lot A contenant des souches isolées de ***Candida albicans***,
- le lot B contenant des souches isolées de ***Candida glabrata***,
- le lot C contenant des souches isolées de ***Candida krusei***,
- le lot D contenant des souches non isolées de divers ***Candida*** (i.e. des souches de ***Candida albicans*** associées à d'autres souches de ***Candida***), et
- le lot témoin étant constitué par ledit lait clarifié et stérilisé.

Après centrifugation à 3500-4000 g pendant 20 minutes à RT, puis culture *in situ* à 37°C pendant 1-2h, on a constaté que le système gélifié de l'exemple 7 donnait une réaction colorée positive avec les tubes des lots A et D et ne donnait aucune réaction colorée positive avec les lots B et C et le lot témoin.

## Exemple 17

Un échantillon d'hémoculture (provenant d'un patient infecté) préalablement mis en contact avec de la saponine (pour lyser les érythrocytes présents) est introduit à raison de 1 à 3 ml dans un tube de centrifugation garni du système gélifié préparé selon le procédé de l'exemple 4. On centrifuge à 3000-5000 g pendant 15 minutes à RT. Après culture *in situ* à 37°C pendant 1-2 h, on observe une réaction colorée positive dans ledit tube de centrifugation, ceci indiquant la présence de bactéries Gram-négatif dans l'hémoculture.

En variante en introduisant dans la phase de révélation ou dans l'ensemble phase de révélation/phase d'absorption un antibiotique de façon à avoir un jeu de plusieurs tubes contenant chacun un antibiotique différent, on peut apprécier la résistance des bactéries contenues dans des hémocultures vis-à-vis d'antibiotiques usuels.

### Exemple 18

On découpe et dilacère finement de la viande boeuf. Au matériau ainsi obtenu, on ajoute du sérum physiologique et des bactéries ($10^7$ germes/ml de souches de **Staphylococcus epidermidis**). On recueille le surnageant et l'introduit à raison de 1-2 ml dans un tube contenant le système gélifié préparé selon le procédé de l'exemple 2.

On centrifuge à 3000-5000 g pendant 15 minutes à RT culture *in situ* à 37°C pendant 24 h. On observe le changement de couleur à la partie inférieure de la phase de révélation ce qui confirme la présence de bactéries dans l'échantillon testé.

### Exemple 19

Dans un tube en centrifugation en plastique transparent d'une hauteur de 30 mm, qui est pourvu d'un capillaire ($\Phi = 2,2$ mm ; volume = 0,045 ml) et qui est conforme à la figure 10, on introduit successivement en centrifugeant après chaque introduction (1000-3000 g pendant 10-20 minutes à RT) pour tasser chaque couche :

- 0,045 ml d'un gel G 100/milieu de culture aqueux, dans lequel G 100 est à une concentration en poids sec de 0,2 g/ml et a une granulomètrie à l'état gonflé de 103-311 μm, ce milieu contenant de la résazurine, et
- 0,5 ml d'huile de paraffine.

On introduit ensuite un échantillon d'urine d'un patient atteint d'infection urinaire provoquée par des souches d'**Escherichia coli**. Après 20 minutes à 3000-5000 g à RT on observe 1 heure après le début de la centrifugation la coloration de la caractéristique de la présence de bactéries.

La numération des bactéries est faite optiquement (spectromètre, densitomètre ou appareil de MacFARLAND destiné à la mesure de la turbidimétrie), le cas échéant avec des solutions d'urine étalon contenant chacune une quantité connue d'**Escherichia coli**.

On récupère la phase de révélation comme expliqué plus haut au moyen d'un deuxième tube de centrifugation contenant la solution aqueuse de récupération afin de réaliser des antibiogrammes pour apprécier la résistance des bactéries recueillis vis-à-vis de plusieurs antibiotiques.

On obtient des résultats particulièrement efficaces quand on diminue le volume du capillaire 7 jusqu'à 0,02-0,03 μl.

Selon l'invention, on préconise un nécessaire, kit ou trousse de dosage qui comporte au moins

(1) un tube de centrifugation à fond conique 6a contenant un système gélifié constitué, de haut en bas, de la phase de protection 4, la phase d'absorption 2 et de la phase de révélation 1, un tube de centrifugation à fond sphérique 6b contenant un système gélifié constitué, de haut en bas, de la phase de protection 4, de la phase d'absorption 2, de la phase de révélation et de la phase barrière 3, ou un tube de centrifugation à fond capillaire 6c contenant un système gélifié constitué de la phase de révélation sur laquelle repose, le cas échéant, la couche de protection et/ou l'ensemble couche de protection + couche d'absorption ; et/ou,

(2) des flacons contenant les matériaux constitutifs (i.e. le ou les gels, la substance particulaire, la paraffine solide et/ou la paraffine liquide) desdites phases, le réactif induisant une variation de mesure optique détectable en présence de microorganismes, et le cas échéant, le milieu de culture pour le gonflement de ladite phase de révélation 1.

On préconise également l'utilisation du procédé et du système gélifié selon l'invention pour

- détecter la présence ou l'absence de bactéries dans un échantillon de sang ou d'urine, notamment dans le domaine du screening des hémocultures et du screening urinaire;
- identifier une souche de microorganisme dans un échantillon biologique en utilisant au niveau de la phase de révélation (α) un réactif induisant une variation de mesure optique détectable et (β) un milieu de culture, qui sont spécifiques de ladite souche,

- procéder à la numération des microorganismes présents dans la phase de révélation,
- recueillir la phase de révélation contenant des microorganismes en vue d'étudier ces derniers, ou
- déterminer la résistance de souches de microorganismes vis-à-vis d'un ensemble d'antibiotiques usuels.

## Revendications

1. Procédé de détection de la présence ou de l'absence de microorganismes appartenant à l'ensemble des bactéries et des levures dans un échantillon liquide (5) d'un matériau biologique susceptible de contenir des cellules autres que celles desdits microorganismes, ledit procédé, qui met en oeuvre une technique de séparation sur gel, étant caractérisé en ce qu'il comprend les étapes consistant à :

   (1°) faire appel à un système gélifié (10) qui (i) sépare en fonction de leur taille les microorganismes, éventuellement présents dans ledit échantillon liquide (5) et qui proviennent dudit matériau biologique, des autres composants solides que peut contenir ledit échantillon liquide, (ii) est essentiellement imperméable à l'eau pouvant être présente dans ledit échantillon liquide et aux substances dissoutes éventuellement présentes dans ledit échantillon liquide, et (iii) comporte au moins

      (a) une première phase (1), dite de révélation, qui est un gel comportant un milieu de culture de microorganismes et un réactif induisant une variation de mesure optique détectable en présence de microorganismes, ledit gel étant un mélange intime d'eau et de particules polymériques absorbant l'eau, qui ont été gonflées de telle façon que dans ledit mélange intime lesdites particules polymériques aient (α) une concentration en poids sec comprise entre 0,05 et 0,2 g/ml et (β) un diamètre à l'état gonflé compris entre 90 et 320 µm, l'eau dudit mélange intime provenant en totalité ou en partie dudit milieu de culture ;

      (2°) introduire ledit échantillon liquide (5) dans un tube de centrifugation (6a, 6b) au-dessus dudit système gélifié (10) préalablement disposé dans ledit tube de centrifugation ;
      (3°) centrifuger le contenu dudit tube (6a, 6b) résultant ; et,
      (4°) révéler la présence ou l'absence de microorganismes dans ledit échantillon liquide (5) provenant dudit matériau biologique, au niveau de ladite première phase (1) du système gélifié (10), par ledit réactif induisant une variation de mesure optique détectable.

2. Procédé suivant la revendication 1, caractérisé en ce que la révélation de l'étape (4°) intervient après multiplication des microorganismes sur ledit milieu de culture, à une température comprise entre la température ambiante et 45°C, en au plus 24 h (de préférence en 1-2 h) pour les bactéries et en au plus 36 h (de préférence en 24-30 h) pour les levures.

3. Procédé suivant la revendication 1, caractérisé en ce que la centrifugation de l'étape (3°) est réalisée à 1000-5000 g.

4. Procédé suivant la revendication 1, caractérisé en ce que, à l'étape (1°), ledit système gélifié comporte en outre

   (b) une seconde phase (2), dite d'absorption, essentiellement imperméable à l'eau pouvant être présente dans ledit échantillon liquide et aux substances qui y sont dissoutes, qui retient dans sa masse les particules non solubles contenues dans ledit échantillon liquide et ayant une taille inférieure à 5 µm mais laisse passer les particules ayant une taille supérieure ou égale à 5 µm lors de la centrifugation, et qui est constituée (i) d'un gel ou (ii) de particules de silice ou de silicate ayant une granulométrie inférieure à 0,1 µm, ladite seconde phase reposant sur ladite première phase, ledit mélange intime étant essentiellement imperméable à l'eau pouvant être présente dans ledit échantillon liquide.

5. Procédé suivant la revendication 4, caractérisé en ce que ladite seconde phase (2) est un gel constitué par un mélange intime d'eau et de particules polymériques absorbant l'eau qui ont été gonflées de telle façon que dans ledit mélange intime lesdites particules polymériques aient (α) une concentration en poids sec comprise entre 0,2 et 1 g/ml et (β) un diamètre à l'état gonflé compris entre 160 et 530 µm.

6. Procédé suivant la revendication 4, caractérisé en ce que ladite seconde phase (2) est une couche constituée d'une matière minérale micronisée de façon à avoir à l'état sec une granulométrie inférieure à 0,1 µm, d'une part, et ne gonflant essentiellement pas en présence d'eau, d'autre part.

7. Procédé suivant la revendication 6, caractérisé en ce que ladite matière minérale est choisie parmi l'ensemble constitué par la silice et les silicates, notamment le kaolin.

8. Procédé suivant la revendication 4, caractérisé en ce que ladite seconde phase (2) renferme une ou plusieurs substances (i) inhibant les éventuels contaminants, (ii) lysant les cellules et/ou (iii) éliminant les lipides, qui sont contenus dans ledit échantillon liquide (5) provenant dudit matériau biologique et susceptibles d'interférer à l'étape (4°) en réagissant avec ledit réactif induisant une variation de mesure optique détectable en présence de microorganismes.

9. Procédé suivant la revendication 1, caractérisé en ce que, à l'étape (1°), ledit système gélifié comporte en outre

   (c) une troisième phase (3), dite barrière, infranchissable lors de la centrifugation de l'étape (3°), par les microorganismes éventuellement présents, ladite troisième phase étant disposée sous la première phase (1) de telle façon que ladite première phase (1) repose sur ladite troisième phase (3).

10. Procédé suivant la revendication 9, caractérisé en ce que ladite troisième phase (3) est une paraffine solide fondant à une température supérieure à la température de multiplication des microorganismes.

11. Procédé suivant la revendication 9, caractérisé en ce que ladite troisième phase (3) est un gel constitué par un mélange intime d'eau et de particules polymériques absorbant l'eau, qui ont été gonflées de telle façon que dans ledit mélange intime lesdites particules polymériques aient ($\alpha$) une concentration en poids sec comprise entre 0,04 et 0,2 g/ml et ($\beta$) un diamètre à l'état gonflé compris entre 30 et 130 $\mu$m.

12. Procédé suivant la revendication 1, caractérisé en ce que, à l'étape (1°), ledit système gélifié comporte en outre

   (d) une quatrième phase (4), dite de protection, qui (i) est une huile inerte de densité inférieure à celle de l'eau, de préférence une huile de paraffine, (ii) avant centrifugation, repose sur ($\alpha$) ladite première phase (1) en l'absence de la seconde phase (2), ou ($\beta$) ladite seconde phase (2) quand celle-ci est présente dans ledit système gélifié (10), et (iii) après centrifugation selon l'étape (3°), repose sur l'eau dudit échantillon liquide (5).

13. Procédé suivant l'une quelconque des revendications 1 et 4 à 12, caractérisé en ce que, à l'étape (1°), on fait appel

   (i) à un système gélifié comprenant, de haut en bas, dans un tube de centrifugation à fond conique (6a) :

   - ladite quatrième phase (4),
   - ladite seconde phase (2), et
   - ladite première phase (1) ;

   (ii) à un système gélifié comprenant, de haut en bas, dans un tube de centrifugation à fond rond ou sphérique (6b) :

   - ladite quatrième phase (4),
   - ladite seconde phase (2),
   - ladite première phase (1), et
   - ladite troisième phase (3) ;

   (iii) à un système gélifié comprenant dans un tube de centrifugation à fond pourvu d'un capillaire 6c : ladite première phase (1) sur laquelle repose, le cas échéant, ladite quatrième phase (4) et/ou ladite seconde phase (2) ; où ladite première phase (1) a un volume plus faible que ceux de ladite seconde phase (2) et dudit échantillon liquide (5) pour concentrer les microorganismes lors de la centrifugation.

14. Système gélifié (10) utilisable suivant le procédé de la revendication 1, ledit système gélifié, étant caractérisé en ce qu'il comporte au moins

   (a) une première phase (1), dite de révélation, qui est un gel comportant un milieu de culture de microorganismes et un réactif induisant une variation de mesure optique détectable en présence de microorganismes, ledit gel étant un mélange intime d'eau et de particules polymériques absorbant l'eau, qui ont été gonflées de telle façon que dans ledit mélange intime lesdites particules polymériques aient (a) une concentration en poids sec comprise entre 0,05 et 0,2 g/ml et (β) un diamètre à l'état gonflé compris entre 90 et 320 μm, l'eau dudit mélange intime provenant en totalité ou en partie dudit milieu de culture.

**15.** Système gélifié suivant la revendication 14, caractérisé en ce qu'il comporte en outre

(b) une seconde phase (2), dite d'absorption, essentiellement imperméable à l'eau pouvant être présente dans ledit échantillon liquide et aux substances qui y sont dissoutes, qui retient dans sa masse les particules non solubles contenues dans ledit échantillon liquide et ayant une taille inférieure à 5 μm mais laisse passer les particules ayant une taille supérieure ou égale à 5 μm lors de la centrifugation, et qui est constituée (i) d'un gel ou (ii) de particules de silice ou de silicate ayant une granulométrie inférieure à 0,1 μm, ladite seconde phase reposant sur ladite première phase, ledit mélange intime étant essentiellement imperméable à l'eau pouvant être présente dans ledit échantillon liquide.

**16.** Système gélifié suivant la revendication 15, caractérisé en ce que ladite seconde phase (2) est un gel constitué par un mélange intime d'eau et de particules polymériques absorbant l'eau qui ont été gonflées de telle façon que dans ledit mélange intime lesdites particules polymériques aient (α) une concentration en poids sec comprise entre 0,2 et 1 g/ml et (β) un diamètre à l'état gonflé compris entre 160 et 530 μm.

**17.** Système gélifié suivant la revendication 15, caractérisé en ce que ladite seconde phase (2) est une couche constituée d'une matière minérale micronisée de façon à avoir à l'état sec une granulométrie inférieure à 0,1 μm, d'une part, et ne gonflant essentiellement pas en présence d'eau, d'autre part.

**18.** Système gélifié suivant la revendication 14, caractérisé en ce qu'il comporte en outre

(c) une troisième phase (3), dite barrière, infranchissable après centrifugation, par les microorganismes éventuellement présents, ladite troisième phase étant disposée sous la première phase (1) de telle façon que ladite première phase (1) repose sur ladite troisième phase (3).

**19.** Système gélifié suivant la revendication 18, caractérisé en ce que ladite troisième phase (3) est une paraffine solide fondant a une température supérieure à la température de multiplication des microorganismes.

**20.** Système gélifié suivant la revendication 18, caractérisé en ce que ladite troisième phase est un gel constitué par un mélange intime d'eau et de particules polymériques absorbant l'eau, qui ont été gonflées de telle façon que dans ledit mélange intime lesdites particules polymériques aient (α) une concentration en poids sec comprise entre 0,04 et 0,2 g/ml et (β) un diamètre à l'état gonflé compris entre 30 et 130 μm.

**21.** Système gélifié suivant la revendication 18, caractérisé en ce qu'il comporte en outre

(d) une quatrième phase (4), dite de protection, qui (i) est une huile inerte de densité inférieure à celle de l'eau, de préférence une huile de paraffine, (ii) avant centrifugation, repose sur (α) ladite première phase (1) en l'absence de la seconde phase (2), ou (β) ladite seconde phase (2) quand celle-ci est présente dans ledit système gélifié (10), et (iii) après centrifugation, repose sur l'eau dudit échantillon liquide (5).

**22.** Tube de centrifugation pourvu à son fond d'un capillaire, utile pour mettre en oeuvre le procédé suivant l'une quelconque des revendications 1 à 13, caractérisé en ce que ledit tube comporte une surface interne tronconique 20 reliant la partie cylindrique du tube au capillaire, l'angle a du cône étant inférieur à 30°, et en ce que la phase de révélation est logée dans la totalité du capillaire (7).

**23.** Tube de centrifugation selon la revendication 22 permettant la numération des bactéries présentes dans la phase de révélation et/ou la récupération de ladite phase de révélation.

**24.** Nécessaire de dosage, utile dans la mise en oeuvre du procédé suivant l'une quelconque des revendications 1 à 13, caractérisé en ce qu'il comporte au moins

(A) un tube de centrifugation à fond conique (6a) contenant un système gélifié constitué, de haut en bas, de la phase de protection (4), la phase d'absorption (2) et de la phase de révélation (1), un tube de centrifugation à fond sphérique (6b) contenant un système gélifié constitué, de haut en bas, de la phase de protection (4), de la phase d'absorption (2), de la phase de révélation (1) et de la phase barrière (3), ou un tube de centrifugation pourvu à son fond d'un capillaire (6c) contenant un système gélifié constitué de la phase de révélation (1) logée dans le capillaire (7), une phase de protection (4) et/ou une phase d'absorption (2) reposant sur ladite phase de révélation (1) ; et/ou

(B) des flacons contenant les matériaux constitutifs desdites phases, le réactif induisant une variation de mesure optique détectable en présence de microorganismes, et au moins le milieu de culture pour le gonflement de ladite phase de révélation (1).

**25.** Utilisation du procédé suivant l'une quelconque des revendications 1 à 13, ladite utilisation étant caractérisée en ce qu'elle comprend

- la détection de la présence ou de l'absence de bactéries dans un échantillon de sang ou d'urine, notamment dans le domaine du screening des hémocultures et du screening urinaire ;
- l'identification d'une souche de microorganisme dans un échantillon biologique en utilisant au niveau de la phase de révélation (α) un réactif induisant une variation de mesure optique détectable et (β) un milieu de culture, qui sont spécifiques de ladite souche,
- la numération des microorganismes présents dans la phase de révélation,
- le recueil de la phase de révélation contenant des microorganismes en vue d'étudier ces derniers, ou
- déterminer la résistance de souches de microorganismes vis-à-vis d'un ensemble d'antibiotiques usuels.

**Patentansprüche**

1. Verfahren zum Nachweis der Gegenwart oder Abwesenheit von Mikroorganismen, die zu Bakterien und Pilzen gehören, in einer flüssigen Probe (5) eines biologischen Materials, das auch andere Zellen als die genannten Mikroorganismen enthalten kann, wobei das Verfahren, das nach einem Trennverfahren auf einem Gel durchgeführt wird, dadurch gekennzeichnet ist, daß es die folgenden Stufen umfaßt:

   (1) Verwenden eines Gelsystems (10), das (i) die Mikroorganismen, die gegebenenfalls in der flüssigen Probe (5) vorhanden sind und aus dem biologischen Material stammen, von anderen festen Komponenten, die in der flüssigen Probe enthalten sein können, nach ihrer Größe trennt, (ii) gegenüber Wasser, daß in der flüssigen Probe enthalten sein kann, und gegenüber anderen gelösten Stoffen, die gegebenenfalls in der flüssigen Probe vorhanden sind, im wesentlichen undurchlässig ist, und (iii) mindestens aufweist

   (a) eine erste Phase (1), Bestimmungsphase genannt, die ein Gel ist, das ein Mikroorganismenkulturmedium und ein Reagens trägt, das in Gegenwart von Mikroorganismen eine Veränderung des optischen Maßes induziert, und das Gel eine innige Mischung aus Wasser und wasserabsorbierenden polymeren Teilchen ist, die so gequollen sind, daß in der innigen Mischung die polymeren Teilchen besitzen (α) eine Konzentration, bezogen auf das Trockengewicht, zwischen 0,05 und 0,2 g/ml, und (β) einen Durchmesser im gequollenen Zustand zwischen 90 und 320 μm, und das Wasser der innigen Mischung vollkommen oder teilweise aus dem Kulturmedium stammt;

   (2) Einbringen der flüssigen Probe (5) in ein Zentrifugenglas (6a,6b) über dem Gelsystem (10) vor Anbringen des Zentrifugenglases;
   (3) Zentrifugieren des Inhalts des Glases (6a,6b); und
   (4) Bestimmen der Gegenwart oder Abwesenheit von Mikroorganismen in der aus dem biologischen Material stammenden flüssigen Probe (5) auf der ersten Phase (1) des Gelsystems (10) mittels des Reagens, das eine Veränderung des optischen Maßes induziert.

2. Verfahren nach Anspruch 1, dadurch gekennzeichnet, daß die Bestimmung nach Stufe (4) nach der Vermehrung der Mikroorganismen auf dem Kulturmedium bei einer Temperatur zwischen Umgebungstemperatur und 45 °C während bis zu 24 Stunden (vorzugsweise 1 bis 2 Stunden) für die Bakterien und bis zu 36 Stunden (vorzugsweise 24 bis 30 Stunden) für die Pilze durchgeführt wird.

3. Verfahren nach Anspruch 1, dadurch gekennzeichnet, daß das Zentrifugieren nach Stufe (3) bei 1000 bis 5000 g durchgeführt wird.

4. Verfahren nach Anspruch 1, dadurch gekennzeichnet, daß in Stufe (1) das Gelsystem außerdem aufweist:

(b) eine zweite Phase (2), Absorptionsphase genannt, die gegenüber Wasser, das in der flüssigen Probe enthalten sein kann, und Substanzen, die in ihm gelöst sein können, im wesentlichen undurchlässig ist, und nichtlösliche Teilchen, die in der flüssigen Probe enthalten sind und eine Größe von kleiner als 5 µm besitzen, zurückhält, aber Teilchen mit einer Größe von größer oder gleich 5 µm bei der Zentrifugation hindurchgehen läßt, und die besteht aus (i) einem Gel oder (ii) Siliciumdioxid- oder Silicatteilchen mit einer Korngröße von kleiner als 0,1 µm, wobei die zweite Phase auf der ersten Phase liegt, und die innige Mischung gegenüber Wasser, das in der flüssigen Probe enthalten sein kann, im wesentlichen undurchlässig ist.

5.  Verfahren nach Anspruch 4, dadurch gekennzeichnet, daß die zweite Phase (2) ein Gel ist, das aus einer innigen Mischung aus Wasser und wasserabsorbierenden Polymerteilchen besteht, die so gequollen sind, daß die polymeren Teilchen in der innigen Mischung besitzen ($\alpha$) eine Konzentration, bezogen auf das Trockengewicht, zwischen 0,2 und 1 g/ml, und ($\beta$) einen Durchmesser im gequollenen Zustand zwischen 160 und 530 µm.

6.  Verfahren nach Anspruch 4, dadurch gekennzeichnet, daß die zweite Phase (2) eine Schicht aus einem mineralischen Material ist, daß so mikronisiert ist, daß es im trockenen Zustand eine Korngröße von kleiner als 0,1 um besitzt, und in Gegenwart von Wasser im wesentlichen nicht quillt.

7.  Verfahren nach Anspruch 6, dadurch gekennzeichnet, daß das mineralische Material ausgewählt ist aus der Gruppe bestehend aus Siliciumdioxid und Silicaten, insbesondere Kaolin.

8.  Verfahren nach Anspruch 4, dadurch gekennzeichnet, daß die zweite Phase (2) eine oder mehrere Substanzen enthält, die (i) gegebenenfalls vorhandene Verunreinigungen inhibieren, (ii) die Zellen lösen, und/oder (iii) die Lipide eliminieren, die in der flüssigen Probe (5) aus dem biologischen Material enthalten sind, und sich der Stufe (4) überlagern können, indem sie mit dem Reagens, das in Gegenwart von Mikroorganismen eine Veränderung des optischen Maßes induziert, reagieren.

9.  Verfahren nach Anspruch 1, dadurch gekennzeichnet, daß in Stufe (1) das Gelsystem außerdem aufweist:

    (c) eine dritte Phase (3), Grenzphase genannt, die bei der Zentrifugation nach Stufe (3) für gegebenenfalls vorhandene Mikroorganismen unüberwindlich ist, und die dritte Phase unter der ersten Phase (1) so angeordnet ist, daß die erste Phase (1) auf der dritten Phase (3) liegt.

10. Verfahren nach Anspruch 9, dadurch gekennzeichnet, daß die dritte Phase (3) ein festes Paraffin ist, das bei einer Temperatur oberhalb der Temperatur der Vermehrung der Mikroorganismen schmilzt.

11. Verfahren nach Anspruch 9, dadurch gekennzeichnet, daß die dritte Phase (3) ein Gel ist, das aus einer innigen Mischung aus Wasser und wasserabsorbierenden polymeren Teilchen besteht, die so gequollen sind, daß die polymeren Teilchen in der innigen Mischung besitzen ($\alpha$) eine Konzentration, bezogen auf das Trockengewicht, zwischen 0,04 und 0,2 g/ml, und ($\beta$) einen Durchmesser im gequollen Zustand zwischen 30 und 130 µm.

12. Verfahren nach Anspruch 1, dadurch gekennzeichnet, daß in Stufe (1) das Gelsystem außerdem aufweist:

    (d) eine vierte Phase (4), Schutzphase genannt, die (i) ein inertes Öl ist, das eine Dichte besitzt, die geringer ist als die von Wasser, und vorzugsweise ein Paraffinöl, (ii) vor der Zentrifugation ($\alpha$) in Abwesenheit der zweiten Phase (2) auf der ersten Phase (1) liegt, oder ($\beta$) auf der zweiten Phase (2) liegt, wenn diese in dem Gelsystem (10) vorhanden ist, und (iii) nach der Zentrifugation gemäß Stufe (3) auf dem Wasser der flüssigen Probe (5) liegt.

13. Verfahren nach einem der Ansprüche 1 und 4 bis 12, dadurch gekennzeichnet, daß man in Stufe (1) verwendet:

    (i) ein Gelsystem, das, von oben nach unten, in einem Zentrifugenglas mit einem konischen Boden (6a) umfaßt:

    -   die vierte Phase (4);
    -   die zweite Phase (2), und
    -   die erste Phase (1);

    (ii) ein Gelsystem, das, von oben nach unten, in einem Zentrifugenglas mit rundem oder kugelförmigen Boden (6b) umfaßt:

- die vierte Phase (4);
- die zweite Phase (2);
- die erste Phase (1), und
- die dritte Phase (3);

(iii) ein Gelsystem, das in einem Zentrifugenglas mit einem mit einer Kapillare ausgestatteten Boden (6c) umfaßt:

die erste Phase (1), auf der gegebenenfalls die vierte Phase (4) und/oder die zweite Phase (2) liegt; und die erste Phase (1) ein viel geringeres Volumen als die zweite Phase (2) und die flüssige Probe (5) besitzt, um die Mikroorganismen bei der Zentrifugation zu konzentrieren.

14. Gelsystem (10) zur Verwendung in einem Verfahren nach Anspruch 1, wobei das Gelsystem dadurch charakterisiert ist, daß es mindestens aufweist

(a) eine erste Phase (1), Bestimmungsphase genannt, die ein Gel ist, das ein Mikroorganismenkulturmedium und ein Reagens trägt, das in Gegenwart von Mikroorganismen eine Veränderung des optischen Maßes induziert, und das Gel eine innige Mischung aus Wasser und wasserabsorbierenden polymeren Teilchen ist, die so gequollen sind, daß in der innigen Mischung die polymeren Teilchen besitzen ($\alpha$) eine Konzentration, bezogen auf das Trockengewicht, zwischen 0,05 und 0,2 g/ml, und ($\beta$) einen Durchmesser im gequollenen Zustand zwischen 90 und 320 µm, und das Wasser der innigen Mischung vollkommen oder teilweise aus dem Kulturmedium stammt.

15. Gelsystem nach Anspruch 14, dadurch gekennzeichnet, daß es außerdem aufweist

(b) eine zweite Phase (2), Absorptionsphase genannt, die gegenüber Wasser, das in der flüssigen Probe enthalten sein kann, und Substanzen, die in ihm gelöst sein können, im wesentlichen undurchlässig ist, und nichtlösliche Teilchen, die in der flüssigen Probe enthalten sind und eine Größe von kleiner als 5 µm besitzen, zurückhält, aber Teilchen mit einer Größe von größer oder gleich 5 µm bei der Zentrifugation hindurchgehen läßt, und die besteht aus (i) einem Gel oder (ii) Siliciumdioxid- oder Silicatteilchen mit einer Korngröße von kleiner als 0,1 µm, wobei die zweite Phase auf der ersten Phase liegt, und die innige Mischung gegenüber Wasser, das in der flüssigen Probe enthalten sein kann, im wesentlichen undurchlässig ist.

16. Gelsystem nach Anspruch 15, dadurch gekennzeichnet, daß die zweite Phase (2) ein Gel ist, das aus einer innigen Mischung aus Wasser und wasserabsorbierenden Polymerteilchen besteht, die so gequollen sind, daß die polymeren Teilchen in der innigen Mischung besitzen ($\alpha$) eine Konzentration, bezogen auf das Trockengewicht, zwischen 0,2 und 1 g/ml, und ($\beta$) einen Durchmesser im gequollenen Zustand zwischen 160 und 530 µm.

17. Gelsystem nach Anspruch 15, dadurch gekennzeichnet, daß die zweite Phase (2) eine Schicht aus einem mineralischen Material ist, daß so mikronisiert ist, daß es im trockenen Zustand eine Korngröße von kleiner als 0,1 µm besitzt, und in Gegenwart von Wasser im wesentlichen nicht quillt.

18. Gelsystem nach Anspruch 14, dadurch gekennzeichnet, daß es außerdem aufweist

(c) eine dritte Phase (3), Grenzphase genannt, die bei der Zentrifugation für gegebenenfalls vorhandene Mikroorganismen unüberwindlich ist, und die dritte Phase unter der ersten Phase (1) so angeordnet ist, daß die erste Phase (1) auf der dritten Phase (3) liegt.

19. Gelsystem nach Anspruch 18, dadurch gekennzeichnet, daß die dritte Phase (3) ein festes Paraffin ist, das bei einer Temperatur schmilzt die höher ist als die Temperatur der Vermehrung der Mikroorganismen.

20. Gelsystem nach Anspruch 18, dadurch gekennzeichnet, daß die dritte Phase ein Gel ist, das aus einer innigen Mischung aus Wasser und wasserabsorbierenden polymeren Teilchen besteht, die so gequollen sind, daß die polymeren Teilchen in der innigen Mischung besitzen ($\alpha$) eine Konzentration, bezogen auf das Trockengewicht, zwischen 0,04 und 0,2 g/ml und ($\beta$) einen Durchmesser im gequollen Zustand zwischen 30 und 130 µm.

21. Gelsystem nach Anspruch 18, dadurch gekennzeichnet, daß es außerdem aufweist

(d) eine vierte Phase (4), Schutzphase genannt, die (i) ein inertes Öl ist, das eine Dichte besitzt, die geringer ist als die von Wasser, und vorzugsweise ein Paraffinöl, (ii) vor der Zentrifugation ($\alpha$) in Abwesenheit der zweiten Phase (2) auf der ersten Phase (1) liegt, oder ($\beta$) auf der zweiten Phase (2) liegt, wenn diese in dem Gelsystem (10) vorhanden ist, und (iii) nach der Zentrifugation auf dem Wasser der flüssigen Probe (5) liegt.

22. Zentrifugenglas, das an seinem Boden mit einer Kapillare ausgestattet ist, zur Verwendung zur Durchführung des Verfahrens nach einem der Ansprüche 1 bis 13, dadurch gekennzeichnet, daß das Glas eine innere kegelstumpfförmige Oberfläche 20 aufweist, die den zylindrischen Teil des Glases mit der Kapillare verbindet, wobei der Winkel $\alpha$ des Konus kleiner als 30 ° ist, und dadurch, daß sich die Bestimmungsphase vollkommen in der Kapillare (7) befindet.

23. Zentrifugenglas nach Anspruch 22, dadurch gekennzeichnet, daß es das Zählen der in der Bestimmungsphase vorhandenen Bakterien und/oder die Wiedergewinnung der Bestimmungsphase ermöglicht.

24. Testsatz zur Verwendung zur Durchführung des Verfahrens nach einem der Ansprüche 1 bis 13, dadurch gekennzeichnet, daß er mindestens aufweist

(A) ein Zentrifugenglas mit einem konischen Boden (6a), das ein Gelsystem enthält, das, von oben nach unten, besteht aus der Schutzphase (4), der Absorptionsphase (2) und der Bestimmungsphase (1); ein Zentrifugenglas mit einem kugelförmigen Boden (6b), das ein Gelsystem enthält, das, von oben nach unten, besteht aus der Schutzphase (4), der Absorptionsphase (2), der Bestimmungsphase (1) und der Grenzphase (3), oder ein Zentrifugenglas, das an seinem Boden mit einer Kapillare ausgestattet ist (6c) und ein Gelsystem enthält, das besteht aus der Bestimmungsphase (1), die sich in der Kapillare (7) befindet, einer Schutzphase (4) und/oder einer Absorptionsphase (2), die auf der Bestimmungsphase (1) liegen; und/oder
(b) Fläschchen, die die Bestandteile dieser Phasen, das Reagens, das eine Veränderung des optischen Maßes in Gegenwart von Mikroorganismen induziert, und mindestens das Kulturmedium zum Quellen der Bestimmungsphase (1) enthalten.

25. Verwendung eines Verfahrens nach einem der Ansprüche 1 bis 13, wobei die Verwendung dadurch charakterisiert ist, daß sie umfaßt

- das Bestimmen der Gegenwart oder Abwesenheit von Bakterien in einer Blut- oder Urinprobe, insbesondere zum Screenen von Blutkulturen und Urinkulturen;
- das Identifizieren eines Mikroorganismenstammes in der biologischen Probe, indem man in der Bestimmungs-phase ($\alpha$) ein Reagens verwendet, das eine meßbare Veränderung des optischen Maßes induziert, und ($\beta$) ein Kulturmedium, das für diesen Stamm spezifisch ist;
- das Zählen der in der Bestimmungsphase vorhandenen Mikroorganismen;
- das Gewinnen der die Mikroorganismen enthaltenden Bestimmungsphase, um diese zu untersuchen, oder
- die Resistenz der Mikroorganismenstämme gegenüber gebräuchlichen Antibiotika zu bestimmen.

## Claims

1. A method of detecting the presence or absence of microorganisms belonging to the group comprising bacteria and yeasts in a liquid sample (5) of a biological material which may contain cells other than those of said microorganisms, said method, which uses a gel separation technique, being characterized in that it comprises the steps consisting in:

(1°) using a gelled system (10) which (i) separates, according to their size, the microorganisms which may be present in said liquid sample (5), and which originate from said biological material, from the other solid components which may be present in said liquid sample, (ii) is essentially impermeable to the water which may be present in said liquid sample and to the dissolved substances which may be present in said liquid sample, and (iii) comprises at least

(a) a first, so-called development phase (1) which is a gel containing a microorganism culture medium and a reagent which induces a detectable variation in optical measurement in the presence of microorganisms, said gel being an intimate mixture of water and water-absorbing polymer particles which have been swollen so that, in said intimate mixture, said polymer particles have ($\alpha$) a concentration by dry weight of between 0.05 and 0.2 g/ml and (β) a diameter in the swollen state of between 90 and 320 µm, the water of said intimate mixture originating wholly or partly from said culture medium;

(2°) introducing said liquid sample (5) into a centrifuge tube (6a, 6b) on top of said gelled system (10), which has been placed in said centrifuge tube beforehand;

(3°) centrifuging the resulting contents of said tube (6a, 6b); and

(4°) revealing the presence or absence of microorganisms in said liquid sample (5) originating from said biological material, in said first phase (1) of the gelled system (10), by means of said reagent which induces a detectable variation in optical measurement.

2. A method according to claim 1, characterized in that the development of step (4°) is carried out after multiplication of the microorganisms in said culture medium at a temperature between room temperature and 45°C, in at most 24 h (preferably in 1-2 h) in the case of bacteria and in at most 36 h (preferably in 24-30 h) in the case of yeasts.

3. A method according to claim 1, characterized in that the centrifugation of step (3°) is carried out at 1000-5000 g.

4. A method according to claim 1, characterized in that, in step (1°), said gelled system also comprises

(b) a second, so-called absorption phase (2) which is essentially impermeable to the water which may be present in said liquid sample and to the substances dissolved therein, which retains in its bulk the insoluble particles contained in said liquid sample which have a size less than 5 µm, but lets through the particles with a size greater than or equal to 5 µm, during centrifugation, and which consists of (i) a gel or (ii) silica or silicate particles with a size less than 0.1 µm, said second phase resting on said first phase, said intimate mixture being essentially impermeable to the water which may be present in said liquid sample.

5. A method according to claim 4, characterized in that said second phase (2) is a gel consisting of an intimate mixture of water and water-absorbing polymer particles which have been swollen so that, in said intimate mixture, said polymer particles have (a) a concentration by dry weight of between 0.2 and 1 g/ml and (β) a diameter in the swollen state of between 160 and 530 µm.

6. A method according to claim 4, characterized in that said second phase (2) is a layer consisting of a mineral substance which on the one hand is micronized so as to have a particle size less than 0.1 µm in the dry state, and on the other hand does not essentially swell in the presence of water.

7. A method according to claim 6, characterized in that said mineral substance is selected from the group comprising silica and silicates, especially kaolin.

8. A method according to claim 4, characterized in that said second phase (2) contains one or more substances which (i) inhibit any contaminants, (ii) lyze the, cells and/or (iii) eliminate the lipids which are contained in said liquid sample (5) originating from said biological material and may interfere in step (4°) by reacting with said reagent which induces a detectable variation in optical measurement in the presence of microorganisms.

9. A method according to claim 1, characterized in that, in step (1°), said gelled system also comprises

(c) a third, so-called barrier phase (3) which cannot be crossed, during the centrifugation of step (3°), by any microorganisms present, said third phase being located underneath the first phase (1) so that said first phase (1) rests on said third phase (3).

10. A method according to claim 9, characterized in that said third phase (3) is a solid paraffin melting at a temperature above the multiplication temperature of the microorganisms.

11. A method according to claim 9, characterized in that said third phase (3) is a gel consisting of an intimate mixture of water and water-absorbing polymer particles which have been swollen so that, in said intimate mixture, said polymer particles have (a) a concentration by dry weight of between 0.04 and 0.2 g/ml and (β) a diameter in the swollen state of between 30 and 130 µm.

12. A method according to claim 1, characterized in that, in step (1°), said gelled system also comprises

(d) a fourth, so-called protection phase (4) which (i) is an inert oil with a density less than that of water, preferably a paraffin oil, (ii) before centrifugation, rests on ($\alpha$) said first phase (1) in the absence of the second phase (2), or ($\beta$) said second phase (2) when the latter is present in said gelled system (10), and (iii) after centrifugation according to step (3°), rests on the water of said liquid sample (5).

13. A method according to any one of claims 1 and 4 to 12, characterized in that the following are used in step (1°):

(i) a gelled system comprising, from top to bottom, in a conical-bottomed centrifuge tube (6a):

- said fourth phase (4),
- said second phase (2) and
- said first phase (1);

(ii) a gelled system comprising, from top to bottom, in a round-bottomed or spherical-bottomed centrifuge tube (6b):

- said fourth phase (4),
- said second phase (2),
- said first phase (1) and
- said third phase (3); or

(iii) a gelled system comprising, in a centrifuge tube 6c, the bottom of which is provided with a capillary: said first phase (1) on which said fourth phase (4) and/or said second phase (2) rest, if appropriate,

the volume of said first phase (1) being smaller than those of said second phase (2) and said liquid sample (5) in order to concentrate the microorganisms during centrifugation.

14. A gelled system (10) which can be used by the method of claim 1, said gelled system being characterized in that it comprises at least

(a) a first, so-called development phase (1) which is a gel containing a microorganism culture medium and a reagent which induces a detectable variation in optical measurement in the presence of microorganisms, said gel being an intimate mixture of water and water-absorbing polymer particles which have been swollen so that, in said intimate mixture, said polymer particles have ($\alpha$) a concentration by dry weight of between 0.05 and 0.2 g/ml and ($\beta$) a diameter in the swollen state of between 90 and 320 $\mu$m, the water of said intimate mixture originating wholly or partly from said culture medium.

15. A gelled system according to claim 14, characterized in that it also comprises

(b) a second, so-called absorption phase (2) which is essentially impermeable to the water which may be present in said liquid sample and to the substances dissolved therein, which retains in its bulk the insoluble particles contained in said liquid sample which have a size less than 5 $\mu$m, but lets through the particles with a size greater than or equal to 5 $\mu$m, during centrifugation, and which consists of (i) a gel or (ii) silica or silicate particles with a size less than 0.1 $\mu$m, said second phase resting on said first phase, said intimate mixture being essentially impermeable to the water which may be present in said liquid sample.

16. A gelled system according to claim 15, characterized in that said second phase (2) is a gel consisting of an intimate mixture of water and water-absorbing polymer particles which have been swollen so that, in said intimate mixture, said polymer particles have ($\alpha$) a concentration by dry weight of between 0.2 and 1 g/ml and. ($\beta$) a diameter in the swollen state of between 160 and 530 $\mu$m.

17. A gelled system according to claim 15, characterized in that said second phase (2) is a layer consisting of a mineral substance which on the one hand is micronized so as to have a particle size less than 0.1 $\mu$m in the dry state, and on the other hand does not essentially swell in the presence of water.

18. A gelled system according to claim 14, characterized in that it also comprises

(c) a third, so-called barrier phase (3) which cannot be crossed, after centrifugation, by any microorganisms present, said third phase being located underneath the first phase (1) so that said first phase (1) rests on said third phase (3).

**19.** A gelled system according to claim 18, characterized in that said third phase (3) is a solid paraffin melting at a temperature above the multiplication temperature of the microorganisms.

**20.** A gelled system according to claim 18, characterized in that said third phase is a gel consisting of an intimate mixture of water and water-absorbing polymer particles which have been swollen so that, in said intimate mixture, said polymer particles have ($\alpha$) a concentration by dry weight of between 0.04 and 0.2 g/ml and ($\beta$) a diameter in the swollen state of between 30 and 130 $\mu$m.

**21.** A gelled system according to claim 18, characterized in that it also comprises

(d) a fourth, so-called protection phase (4) which (i) is an inert oil with a density less than that of water, preferably a paraffin oil, (ii) before centrifugation, rests on ($\alpha$) said first phase (1) in the absence of the second phase (2), or ($\beta$) said second phase (2) when the latter is present in said gelled system (10), and (iii) after centrifugation, rests on the water of said liquid sample (5).

**22.** A centrifuge tube provided with a capillary at the bottom, which is useful for carrying out the method according to any one of claims 1 to 13, characterized in that said tube has a frustoconical inner surface 20 joining the cylindrical part of the tube to the capillary, the angle $\alpha$ of the cone being less than 30°, and in that the development phase is accommodated in the whole of the capillary (7).

**23.** A centrifuge tube according to claim 22 which makes it possible to count the bacteria present in the development phase and/or recover said development phase.

**24.** An assay kit which is useful in carrying out the method according to any one of claims 1 to 13, characterized in that it comprises at least

(A) One conical-bottomed centrifuge tube (6a) containing a gelled system consisting, from top to bottom, of the protection phase (4), the absorption phase (2) and the development phase (1), a spherical-bottomed centrifuge tube (6b) containing a gelled system consisting, from top to bottom, of the protection phase (4), the absorption phase (2), the development phase (1) and the barrier phase (3), or a capillary-bottomed centrifuge tube (6c) containing a gelled system consisting of the development phase (1) accommodated in the capillary (7), a protection phase (4) and/or an absorption phase (2) resting on said development phase (1); and/or
(B) bottles containing the constituent materials of said phases, the reagent which induces a detectable variation in optical measurement in the presence of microorganisms, and at least the culture medium for the swelling of said development phase (1).

**25.** A use of the method according to any one of claims 1 to 13, said use being characterized in that it comprises

- detecting the presence or absence of bacteria in a blood or urine sample, especially in the field of hemoculture screening and urine screening,
- identifying a strain of microorganism in a biological sample by using, in the development phase, ($\alpha$) a reagent which induces a detectable variation in optical measurement, and ($\beta$) a culture medium, both of which are specific for said strain,
- counting the microorganisms present in the development phase,
- collecting the development phase containing microorganisms for the purpose of studying the latter, or
- determining the resistance of strains of microorganisms towards a group of customary antibiotics.

Fig. 1

Fig. 2a

Fig. 2b

Fig. 3

Fig. 4a

Fig. 4b

Fig. 5

Fig. 6a

Fig. 6b

Figure 8

Figure 7

Fig. 9

Fig. 10